(19) Europäisches Patentamt
European Patent Office
Office européen des brevets

(11) Publication number: **0 013 617**
**B1**

(12) **EUROPEAN PATENT SPECIFICATION**

(45) Date of publication of patent specification: 04.07.84

(51) Int. Cl.³: C 07 D 499/00, A 61 K 31/43 //C07D513/04

(21) Application number: 80300067.8

(22) Date of filing: 07.01.80

# (54) Penicillin derivatives, process for their preparation and pharmaceutical compositions containing certain of these compounds.

(30) Priority: 10.01.79 GB 7900903
29.03.79 GB 7911038
04.04.79 GB 7911764
03.05.79 GB 7915469

(43) Date of publication of application:
23.07.80 Bulletin 80/15

(45) Publication of the grant of the patent:
04.07.84 Bulletin 84/27

(84) Designated Contracting States:
BE CH DE FR GB IT NL SE

(56) References cited:
EP - A - 0 005 889
GB - A - 956 605
US - A - 4 123 539

ACTA CHEMICA SCANDINAVICA, Series B, vol. B 30, no. 5, 1976, pages 377-382 N.G.

JOHANSSON: "Synthesis of Penicillin Analogs, (-)-Methyl 6-epi-6-Bromobisnorpenicillanate and (+,-)-Methyl 6-epi-6-Bromopenicillanate"

(73) Proprietor: BEECHAM GROUP PLC
Beecham House Great West Road
Brentford Middlesex (GB)

(72) Inventor: John, David Ivor
120 Tattenham Grove
Epsom Downs, Surrey (GB)
Inventor: Tyrrell, Nicholas David
Oak Field House Debden Green
Loughton, Essex (GB)
Inventor: Thomas, Eric James
25 Alzey Gardens
Harpenden, Hertfordshire (GB)
Inventor: Giddings, Peter John
28 Orchard Road
Westbury, Wiltshire (GB)
Inventor: Denerley, Paul Millington
12 The Gables Wimblehurst Road
Horsham, Sussex (GB)

(74) Representative: Hesketh, Alan, Dr. et al,
European Patent Attorney Beecham
Pharmaceuticals Great Burgh Yew Tree Bottom
Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 013 617 B1

(56) References cited:

JOURNAL OF ORGANIC CHEMISTRY, vol. 43, no. 18, 1st September 1978, pages 36113613 M.J. LOOSEMORE et al.: "On the Epimerization of 6gamma-Bromopenicillanic Acid and the Preparation of 6beta-Bromopenicillanic Acid"

JOURNAL OF THE CHEMICAL SOCIETY, no. 21, 1st November 1979, pages 962-963 BARRY S.ORLEK et al.: "On the Chemical Binding of 6 beta-Bromopenicillanic Acid to beta-Lactamse I"

**Description**

This invention relates to a process for the preparation of certain penicillin derivatives which are useful as β-lactamase inhibitors. The compounds which may be prepared by the process of this invention have formula (I), or a pharmaceutically acceptable salt or ester thereof:

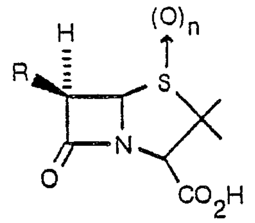

(I)

wherein R represents hydrogen, halogen, $C_{1-6}$alkylthio, $C_{1-6}$alkyl or alkyl substituted with phenyl, carboxy, $C_{1-6}$alkoxycarbonyl, hydroxy or $C_{1-6}$alkylthio, and n is zero, 1 or 2. In addition certain of the compounds produced by the process of the invention are novel compounds.

It is known that 6β-(substituted)alkyl penicillanic acid derivatives may be prepared by hydrogenation of the corresponding 6-(substituted)alkylidene penam which can be obtained via a Wittig reaction on the 6-oxo penam compound (J.C. Sheehan and Y.S. Lo, J. Org. Chem. 1973, *38*, 3227).

A mixture of 6-α and 6-β α-hydroxyethyl penams has been prepared by reducing a 6-bromo, 6-hydroxyethyl penam with zinc/silver (F. DiNinno *et al*, T.R. Beattie, and B.G. Christensen, J. Org. Chem., 1977, *42*, 2961).

Compounds of the formula (I) wherein R is an ethyl group have been prepared by the successive treatment of a compound wherein R is a hydroxyethyl group, with a sulphonating agent, a base and finally catalytic hydrogenation (United States Patent No. 4,123,539).

Compounds of the formula (I) wherein R is a hydrogen atom have been prepared by the reduction of a 6,6-dihalopenicillanate, and in particular 1,1-dioxopenicillanic acid has been stated to possess β-lactamase inhibitory qualities (A.R. English *et al*, Antimicrobial Agents and Chemotherapy, 1978, *14*, 414).

Benzyl 6β-chloropenicillanate has been reported to have been obtained in 3% yield as a by-product of the preparation of benzyl 6β-hydroxypenicillanate from benzyl 6-oxopenicillanate (Roets *et al*, J.C.S. Perkin 1, 1976, 704), but no utility has been suggested for this compound. 6β-Chloropenicillanic acid has been reported not to be an inhibitor of β-lactamase (Cartwright *et al.*, Nature *278*, 22 March 1979, page 360).

It is known that 6-β-bromopenicillanic acid is a β-lactamase inhibitor (see Loosemore *et al*, J. Org. Chem., *43*, 3611 (1978); Pratt *et al.*, Proc. Natl. Acad. Sci. U.S.A., *75*, 4145 (1978); Knott-Hunziker *et al.*, F.E.B.S. Letters, 59, (1979); Orlek *et al.*, J.C.S. Chem. Comm., 1979, 962; and Knott-Hunziker *et al.*, Biochem J., *177*, 365 (1979)). In these references 6-β-bromopenicillanic acid has always been described as a minor component of a mixture together with 6-α-bromopenicillanic acid. The process of the present invention enables substantially pure salts of 6β-bromopenicillanic acid to be prepared.

Furthermore, in vivo hydrolysable esters of the 6-β-halopenicillanic acids may be prepared by the present process. No such esters of 6-β-halopenicillanic acids have yet been described whether pure or in admixture with the corresponding 6-α-compound. These esters are advantageous because the 6-β-bromopenicillanic acid tends to be unstable and is apparently readily isomerised to a mixture with the 6-α-compound. It would therefore be desirable to provide a compound that released 6-β-bromopenicillanic acid in-vivo without significant contamination of the inactive 6-α-compounds.

Accordingly the present invention provides the compounds of the formula (II):

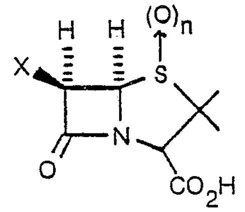

(II)

and pharmaceutically acceptable salts and in-vivo hydrolysable esters thereof wherein X is a bromine or chlorine atom, and n is zero, 1 or 2, when substantially free from the corresponding 6α-substituted compound.

Favourably n in the compound of the formula (II) is zero or 2.

One preferred compound of this invention is the compound of the formula (III):

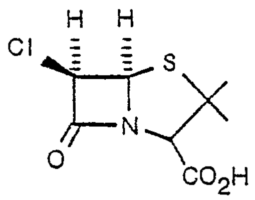

(III)

and pharmaceutically acceptable salts and in-vivo hydrolysable esters thereof.

A further preferred class of compounds are the in-vivo hydrolysable esters of the compound of the formula (IV):

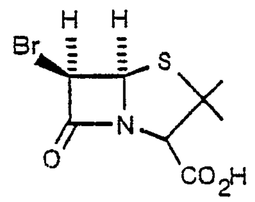

(IV)

More suitably the 6-$\beta$-compound of this invention is not contaminated by more than 10% w/w of the corresponding 6-$\alpha$-compound, favourably is not contaminated by more than 5% w/w of the corresponding 6-$\alpha$-compound and preferably is not contaminated by more than 2% w/w of the corresponding 6-$\alpha$-compound.

The presence of any contaminating 6-$\alpha$-compound may be estimated by standard analytical techniques such as n.m.r. spectroscopy, or h.p.l.c.

The compounds of the formula (II) are suitably in the form of the free acid.

Alternatively the compounds of the formula (II) are in the form of a pharmaceutically acceptable salt such as a metal salt, e.g. aluminum, alkali metal salts such as sodium or potassium, alkaline earth metal salts such as calcium or magnesium, and ammonium or substituted ammonium salts for example those with lower alkylamines such as triethylamine, hydroxy-lower alkylamines such as 2-hydroxyethylamine, bis-(2-hydroxyethyl)-amine, tris (hydroxymethyl)amine or tris(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-$\beta$-phenethylamine, dehydroabietylamine, N,N'-bis- dehydroabietylethylenediamine, or bases of the pyridine type such as pyridine, collidine or quinoline, or other amines which have been used to form salts with penicillins.

Favourably the compounds of the formula (II) are in the form of an alkali or alkaline earth metal salt. Particularly favoured are the compounds of the formula (II) when in the form of an alkali metal salt, for example a sodium or potassium salt. From the foregoing it is to be realised that favoured compounds of this invention include alkali metal salts of 6$\beta$-bromopenicillanic acid, for example sodium 6$\beta$-bromopenicillanate and potassium 6$\beta$-bromopenicillanate.

The compounds of the formulae (II)—(IV) as hereinbefore defined may be provided as in-vivo hydrolysable esters. Such esters are those which hydrolyse in the human body to produce the parent acid. Suitable in-vivo hydrolysable esters include those esters known to give in-vivo hydrolysis in penicillins. Thus suitable esters include those of the sub-formula (a):

$$—CO—O—CHR_1—O—CO—R_2 \qquad (a)$$

wherein $R_1$ is a hydrogen atom or a methyl group; $R_2$ is an alkyl group of 1 to 6 carbon atoms, a phenyl group, an alkyl group of 1 to 3 carbon atoms substituted by a phenyl group, an alkoxyl group of 1 to 6 carbon atoms, a phenoxyl group, or an alkoxyl group of 1 to 3 carbon atoms substituted by a phenyl group; or $R_1$ is attached to $R_2$ to form a 1,2-diphenylene or 4,5-dimethoxy-1,2-diphenylene group.

Favourably $R_1$ is hydrogen.

When $R_1$ is hydrogen suitably $R_2$ is selected from methyl, ethyl, n-propyl, iso-propyl, n-butyl, tert-butyl, phenyl, benzyl, methoxy, ethoxy, n-propyloxyl and iso-propyloxy. Preferably $R_2$ is tert-butyl.

Favourably $R_1$ and $R_2$ are joined so that the ester is a phthalidyl or 3,4-dimethoxyphthalidyl ester.

Thus preferred in-vivo hydrolysable ester groups include acetoxymethyl, pivaloyloxymethyl, $\alpha$-acetoxyethyl, $\alpha$-acetoxybenzyl, $\alpha$-pivaloyloxyethyl, ethoxycarbonyloxymethyl, $\alpha$-ethoxycarbonylethyl, phthalidyl and 5,6-dimethoxyphthalidyl.

Other suitable in-vivo hydrolysable groups include dialkylamino alkyl groups such as dimethyl-

aminomethyl, dimethylaminoethyl, diethylaminomethyl and diethylaminoethyl; and N-phthalimidomethyl and methoxymethyl groups.

The in-vivo hydrolysable nature of the ester may be confirmed by administration to an animal such as a mouse or rat and determination of the presence of 6-β-bromopenicillanate in the blood or urine of the animal. Alternatively hydrolysis in human blood or serum may be determined.

The methoxymethyl ester is also of use as an intermediate for the preparation of salts of the compounds of the formula (I) as described hereinbelow.

The present invention also provides a pharmaceutical composition which comprises a compound of the formula (II) or a pharmaceutically acceptable salt or in-vivo hydrolysable ester as hereinbefore defined and a pharmaceutically acceptable carrier.

The composition of this invention may be adopted for administration by injection or by the oral route.

Generally the composition will be presented as a unit dose containing from 60 to 600 mgs of the β-lactamase inhibitor, more suitably 100 to 500 mgs. of the β-lactamase inhibitor and preferably from 125 to 300 mgs. of the inhibitor. Such compositions may be administered 2—6 times daily and usually 3 or 4 times daily in a manner such that the total daily dose for a 70 kg human will be about 200 to 1000 mgs.

The composition of this invention may be administered concurrently or consecutively with a penicillin or cephalosporin. However, it is greatly preferred to administer the penicillin or cephalosporin in the same composition as the β-lactamase inhibitor of this invention. British Patent Specification No. 1,508,978 discloses suitable penicillins and cephalosporins for use in such synergistic compositions. The forms of compositions, methods of preparation and ratios of components disclosed in Specification No. 1,508,978 may be also used with the synergists of this invention. The disclosures of Specification 1,508,978 with respect to compositions are accordingly incorporated herein by reference. A suitable penicillin is amoxycillin as the trihydrate or sodium salt.

The present invention provides a process for the preparation of a penicillin derivative of formula (I) above or a pharmaceutically acceptable salt or ester thereof, which process comprises treating a compound of formula (V):

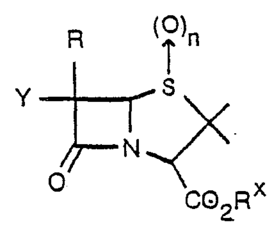

(V)

wherein either (a) R is as defined with respect to formula (I) and Y represents isocyano or halogen, or (b) R is chloro and Y is arylselenyl; and $R^x$ represents hydrogen or a carboxyl-blocking group, and n is zero 1 or 2; with a triaryl tin hydride, dialkyltin hydride or trialkyl tin hydride and thereafter optionally carrying out one or more of the following steps:

(i) converting a sulphide ($n = 0$), sulphoxide ($n = 1$), or sulphone ($n = 2$) to a different such group;
(ii) removing the carboxyl-blocking group if present;
(iii) converting the free penicillanic acid into a pharmaceutically acceptable salt or ester thereof.

The group Y is preferably isocyano (CN-), chlorine, bromine or iodine.

Suitable values for the group R include chlorine, fluorine, bromine, iodine, methylthio, ethylthio, methyl, ethyl, *n*- and *iso*- propyl, *n*-, *sec*- and *iso*butyl, benzyl, phenylethyl, phenylpropyl, carboxymethyl, carboxyethyl, carboxypropyl, methoxycarbonylmethyl, methoxycarbonylethyl, ethoxycarbonylmethyl, ethoxycarbonylethyl, hydroxymethyl, 1- or 2-hydroxyethyl, 1-, 2- or 3-hydroxy-*n* -propyl, 1- or 2-hydroxy-*iso*-propyl, methylthiomethyl, methylthioethyl.

Suitably the hydride is a tri-$C_{1-6}$ alkyl tin hydride.

When used herein the term "aryl" means a phenyl group or a phenyl group substituted by $C_{1-3}$ alkyl, $C_{1-3}$ alkoxy, hydroxy or nitro. A preferred triaryl tin hydride is triphenyl tin hydride. Another preferred reagent is tri-n-butyl tin hydride.

When Y is arylselenyl a preferred group is phenylselenyl.

Suitable carboxyl-blocking derivatives for the group —$CO_2R^x$ in formula (V) include salts and ester derivatives of the carboxylic acid. The derivative is preferably one which may readily be cleaved at a later stage of the reaction. Suitable salts include tertiary amine salts, such as those with tri-loweralkylamines, N-ethylpiperidine, 2,6-lutidine, pyridine, N-methylpyrrolidine, dimethylpiperazine. A preferred salt is with triethylamine.

Suitable ester-forming radicals for the group $R^x$ are esters which are compatible with the use of the tin hydrides and include benzyl, p-methoxybenzyl, 2,4,6-trimethylbenzyl, benzoylmethyl, 4-pyridylmethyl, 2,2,2-trichloroethyl, t-butyl, t-amyl, diphenylmethyl, triphenylmethyl, adamantyl, 2-benzyloxy-

phenyl, 4-methylthiophenyl, tetrahydrofur-2-yl, tetrahydropyran-2-yl, pentachlorophenyl, methoxymethyl, silyl groups such as trimethylsilyl or 5-butyldiphenylsilyl, trialkyltin esters or oxime ester radicals of formula $N=CHR^o$ where $R^o$ is aryl or heterocyclic.

The carboxyl group may be regenerated from any of the above esters by usual methods appropriate to the particular $R^x$ group, for example, acid- and base-catalysed hydrolysis, or by enzymically-catalysed hydrolysis, or by hydrogenation.

Preferably the reduction of this invention is carried out in the presence of a radical initiator. Suitable radical initiators include di-*t*-butyl peroxide and azobisisobutyronitrile.

The reaction is generally operated in an inert solvent, such as benzene, toluene, alkanols, such as ethanol, and saturated hydrocarbons. Halogenated hydrocarbon solvents and ethers are not suitable. Once initiated, the reaction is fairly facile. However, the reaction mixture normally requires heating to about 80°C before much reaction takes place. Once the reaction has started at this temperature, it is usually over within a few minutes, although it is generally preferable to heat the reaction mixture for about an hour to be certain the reaction is complete. At lower temperatures, the reaction may be initiated by using a radical initiator, or photochemically. At higher temperatures the reduction is rapid, but may be accompanied by thermal degradation of the penicillin.

Although trialkyl tin hydrides have been used to remove isocyano groups in the past, for example benzyl isocyanide has been reduced to toluene with tri-*n*-butyl tin hydride (T. Saegusa et al, J.A.C.S. 1968, *90*, 4182), it has not been previously suggested that the reagents could be employed on penicillin derivatives. One major advantage of the reaction of the present invention is that it has considerable stereoselectivity and gives a great preponderence of the 6β-substituted isomer of compound (I).

We have found that the hereinabove described reaction affords tin residues that are difficult to eliminate completely from the products of this reaction; occasionally it has proved necessary to subject a reaction product to rechromatography. We have discovered that the use of an aqueous solution of a fluoride ion, for example aqueous potassium fluoride when stirred with the crude reaction product in an organic solvent such as chloroform, forms a precipitate which may readily be filtered to provide a filtrate containing crude reaction product essentially free of tin residual impurities. This modification has been found only to be an improvement in the process described hereinabove when Y is a halogen atom in the compounds of the formula (V).

The compound (I) resulting after this reduction process may be a sulphide ($n=0$), sulphoxide ($n=1$) or sulphone ($n=2$) at the 1-position. After the reduction these groups may if desired, be converted to a different such group by standard methods of reduction or oxidation.

The intermediate of formula (V) wherein Y represents isocyano may be prepared as described in U.S. Patent No. 3,996,235. The intermediates of formula (V) wherein Y represents halogen may be prepared as described in J. Chem. Soc. (C) 1969, 2123; J. Org. Chem., 1977, *42*, 2961 and J. Org. Chem., 1978, *43*, 2203. The intermediates of formula (V) wherein Y is arylselenyl and R is chloro may be prepared by the reaction of a compound of the formula (VI):

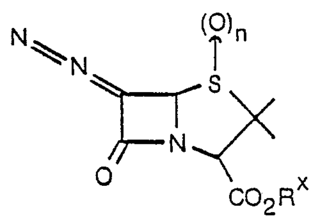

(VI)

wherein n is defined in relation to formula (V), and $R^x$ is a carboxy-blocking ester as defined in relation to formula (V), with an arylselenyl chloride in an inert solvent such as dichloromethane at an ambient temperature.

Compounds of the formula (VI) may be prepared by the methods of J. Org. Chem, 1974, *39*, 1444.

The present invention also provides a process for the preparation of an in-vivo hydrolysable ester of 6-β-bromopenicillanic acid when substantially free from the corresponding in-vivo hydrolysable ester of 6-α-bromopenicillanic acid which process comprises preparing a mixture of isomeric in-vivo hydrolysable esters of 6α- and 6β-bromopenicillanic acids and thereafter chromatographically separating the mixtures of isomers into fractions and recovering the in-vivo hydrolysable ester of 6-β-bromopenicillanic acid from a fraction containing said in-vivo hydrolysable ester of 6-β-bromopenicillanic acid substantially free from the in-vivo hydrolysable ester of a 6-α-bromopenicillanic acid.

The mixture of isomeric in-vivo hydrolysable esters may be prepared by reaction of a mixture of the salts with a reactive halide or the like, for example, with a compound of the formula (VII):

$$X—CHR_1—O—CO—R_2 \qquad (VII)$$

wherein $R_1$ and $R_2$ are as hereinbefore defined in relation to sub-formula (a) and X is Cl, Br or I.

Esterification to afford the dialkylaminoalkyl, N-phthalimidomethyl or methoxymethyl esters is similarly performed, for example using dialkylaminoalkyl halides, N-(chloromethyl) phthalimide or chloromethyl methyl ether respectively.

Alternatively the mixture of isomeric in-vivo hydrolysable esters of 6α- and 6β-bromopenicillanic acid may be prepared by the treatment of an in-vivo hydrolysable ester of 6,6-dibromopenicillanic acid with a strong organic base such as methyl lithium followed by protonation. Protonation is most conveniently effected by acetic acid. The treatment with base is carried out in anhydrous conditions at a low temperature, for example under nitrogen in a dry solvent at −78°C. In vivo hydrolysable esters of 6,6-dibromopenicillanic acid are prepared by conventional methods of esterification.

Such esterification reactions to prepare a mixture of isomeric in-vivo hydrolysable esters of 6α- and 6β-bromopenicillanic acids will be carried out under conditions similar to those known to be suitable for the preparation of in-vivo hydrolysable esters of penicillins. For example the reaction may be carried out in an organic solvent such as dimethylformamide, optionally together with methylene chloride.

The salt of the mixture of 6-bromopenicillanic acids may be generated in situ, for example by using an organic tertiary base such as trimethylamine or triethylamine.

Since known mixtures of 6-bromopenicillanic acids contain only a minor proportion (usually about 12% w/w) of the desired 6-β-bromo isomer, the resulting mixture of in-vivo hydrolysable esters only contains a minor proportion of the desired 6-β-bromo isomer. As will be appreciated by the skilled chemist obtaining a reactive minor component from a mixture of materials is difficult and not necessarily possible. Fortunately it has been found that repeated chromatography of the mixture of in-vivo hydrolysable esters can produce the desired in-vivo hydrolysable ester 6-β-bromo isomer substantially free from the corresponding in-vivo hydrolysable ester of the 6-α-bromo isomer.

Suitable methods of chromatography include column chromatography using silica gel as stationary phase. Gradient elution using ethyl acetate/cyclohexane mixtures has been found suitable. The progress of the individual product through the chromatographic system may be monitored by thin layer chromatography and developing with Ehrlich's reagent. The initial major product is the 6-α-isomer and the initial minor product is the 6-β-isomer so that Rf values and relative positions may be known.

Initial separation of the two isomers is not good so that the leading edge of the faster running component or the trailing edge of the slower running component is selected for rechromatography. Two or three repetitions of this chromatographic process are normally sufficient to produce the desired substantially pure product. In the silica gel ethyl acetate/cyclohexane system described in Example 22 herein the 6-β-isomer is the slower running component so that the trailing edge of the slower running component is selected for rechromatography.

The 6α, β-starting material may be obtained as described in the Loosemore et al., reference referred to hereinbefore.

The following Examples illustrate the processes of this invention:

When referring to the Examples the following should be noted:

Thin layer chromatography (t.l.c.) was carried out on Merck Kieselgel plates containing $F_{254}$, and was used for assessment of purity and for following the progress of reactions. The eluant used was usually ethyl acetate (15—100%) in light petroleum (redistilled b.p. 60—70°C), and plates were visualized by uv, aqueous potassium permanganate or iodine vapour. Column chromatography using similar solvent mixtures was carried out on Hopkin and Williams Silica gel MFC (without binder).

Toluene-*p*-sulphonates were converted to their free bases by washing a suspension of the salt in chilled ethyl acetate with a several fold excess of aqueous sodium bicarbonate and drying.

Unless otherwise stated, all optical rotations were determined for solutions in chloroform, i.r. spectra as thin films and n.m.r's as solutions in deuteriochloroform with tetramethylsilane as internal standard and were determined at 90 MHz. Melting points are uncorrected; DMF is *N,N*-dimethylformamide, ether refers to diethyl ether, EtOAc is ethyl acetate, MIBK is methyl *iso*-butyl ketone (3-methylpentan-2-one), and THF is tetrahydrofuran. ABIBN and SEH are respectively azo bis-isobutyronitrile and sodium ethyl hexanoate (used as a 2 molar solution in MIBK).

### Example 1

Benzyl 6-β-(2′-hydroxyprop-2′-yl)penicillanate

A solution of benzyl 6-α-(2′-hydroxyprop-2′-yl)-6-β-isocyanopenicillanate (prepared by the methods detailed by Bentley and Clayton J.C.S. Chem. Comm 1974 p 278) (0.459 g, 1.22 mmol) in dry benzene (10 cm³) was treated with ABIBN (0.040 g), 20 mol %) followed by tri-*n*-butyl tin hydride (0.375 g, 1.28 mmol), and the mixture heated under reflux (nitrogen atmosphere) for 15 min. After removal of solvent under reduced pressure, fractionation by silica-gel chromatography (3:7 EtOAc-petrol as eluant) gave, in order of elution, the required benzyl 6-β-(2′-hydroxyprop-2′yl)-penicillanate (0.267 g, 63%), and a by-product (1R, 5R)-6-(1-benzyloxycarbonyl-2-methylprop-1-yl)-1-(2-hydroxyprop-2-yl)-4-thia-2,6-diazabicyclo(3.2.0)hept-2-ene-7-one (0.083 mg, 18%), both slightly contaminated with trace amounts of alkyl tin residues.

Rechromatography (silica-gel) of the β-alkylpenicillanate gave a clear oil: $[\alpha]_D^{20} = +123.9°$

($CHCl_3$); $\nu_{max}$ (film) 3460br (—OH), 1775 (lactam C = O), 1745 $cm^{-1}$ (ester C = O); $\delta$ ($CDCl_3$) 1.29, 1.67 (each 3H, s, thiazolene *gem*-$Me_2$), 1.42 (6H, s, alkyl *gem*-$Me_2$), 3.38 (1H, s, —O*H* exchangeable with $D_2O$), 3.64 (1H, d, *J* 4.4Hz, $C_6$*H*), 4.50 (1H, s, $C_3$—*H*), 5.18 (2H, s, Ph—$CH_2$), 5.46 (1H, d, *J* 4.4Hz; $C_5$—*H*), 7.37 (5H, s, Ph—*H*); m/e 349($M^+$), 91($C_7H_7^+$); Accurate mass 349.1358 observed, $C_{18}H_{23}NO_4S$ requires 349.1346.

Recrystallisation (EtOAc-petrol) of the bicyclic byproduct gave pure sample: $[\alpha]_D^{20} = 43.35°$ ($CHCl_3$); $\nu_{max}$ (film) 1765 (lactam C = O), 1735 $cm^{-1}$ (ester C = O); $\delta$ ($CDCl_3$) 0.9, 0.95 (each 3H, d, *J* 8.7Hz, methylpropyl $Me_2$), 1.20, 1.51 (each 3H, s, *iso*-propyl $Me_2$), 2.04 (1H, s, O*H*-exchanges with $D_2O$), 2.4(1H, m, methylpropyl $C_2$—*H*), 4.1(1H, d, *J* 9.1Hz, methylpropyl $C_1$—*H*), 5.17 (2H, ABq, $J_{gem}$12Hz, pH—$CH_2$), 5.78 (1H, d, *J* 1.5Hz, $C_5$—*H*), 7.35 (5H, s, Ph—*H*), 8.01 (1H, d, *J* 1.5Hz, $C_3$—*H*) (Found: C, 60.567; H, 6.308; N, 7.356; S, 8.434, $C_{19}H_{24}N_2O_4S$ requires C, 60.617; H, 6.426; N, 7.441; S, 8.517%); m/e 377 ($M^+$), 91($C_7H_7^+$); m.p. 102.5—103.5°C.

## Example 2

6$\beta$-(1-Hydroxy-1-methylethyl)penicillanic acid

Benzyl 6-$\beta$-(1-hydroxy-1-methylethyl)penicillanate (100 mg, 0.286 mmol) was dissolved in ethyl acetate (5 ml) and hydrogenated at 55 psi over 10% Pd-C catalyst (100 mg) for 1 hour. No change in the nmr of the isolated penam (filtration and evaporation) could be seen beyond broadening of the —OH peak, but after a further 3 hrs of hydrogenation over fresh catalyst this —OH peak had moved downfield in the nmr spectrum. A third period of hydrogenation (75 mins) gave a white, crystalline solid (65 mg, 87%), identified as the title compound. Crystallisation from acetone-petroleum ether gave 37 mg product; $\delta$ ($Me_2CO$-$d_6$) 1.30, 1.37, 1.55, 1.70 (12H, 4s, *gem*-$Me_4$), 3.83 (1H, d, J = 4.6Hz, $H_6$), 4.04 (s, —$CO_2H$ + —OH), 4.33 (1H, s, $H_3$), 5.46 (1H, d, J = 4.6Hz, $H_5$) ppm; $\nu_{max}$ (film) 3380, 1740 and 1735 $cm^{-1}$; m/e observed 259.0862 $C_{11}H_{17}NO_4S$ requires m/e 259.0877.

## Example 3

Benzyl 6-$\beta$-1′-hydroxy-1′-methylethyl)-1,1-dioxopenicillanate

Benzyl 6-$\beta$-(1′-hydroxy-1′-methylethyl)penicillanate (100 mg, 0.265 mmol) was dissolved in chloroform (5 ml), to which solution was added *m*-chloroperbenzoic acid, followed by stirring overnight. Filtration, followed by washing with sodium sulphite, then sodium bicarbonate and drying over $MgSO_4$ gave a mixture of products by nmr. Chromatography yielded the title compound (18 mg, 18%); $\delta$ ($CDCl_3$) 1.30, 1.56 (6H, 2s, *gem*-$Me_2$), 1.43 (6H, s, —C*$Me_2$*OH), 3.85 (1H, s, OH), 3.95 (1H, d, J = 5.5Hz, $H_6$), 4.56 (1H, s, $H_3$), 4.74 (1H, d, J = 5.5Hz, $H_5$), 5.22 (2H, ABq, —$CO_2CH_2$—), 3.36 (5H, s, —$CH_2C_6H_5$) ppm; m/e 259, 124, 91 and 83 prominent.

## Example 4

6-$\beta$-(1′Hydroxy-1′-methylethyl)-1,1-dioxopenicillanic acid

Benzyl 6-$\beta$-(1′-hydroxy-1′-methylethyl)-1,1-dioxopenicillanate (18 mg, 0.047 mmol) was dissolved in ethyl acetate (3 ml) and hydrogenated at 55 psi over 10% Pd-C catalyst (20 mg) for 1 hour. Filtration and evaporation gave the free acid as a solid (10 mg, 73%); $\delta$ ($Me_2CO$-$d_6$)/ppm 1.40 (6H, s, —C*$Me_2$*OH), 1.52, 1.62 (6H, 2s, *gem*-$Me_2$), 4.3 (1H, d, J $\simeq$ 5Hz, $H_6$) 4.5 (1H, s, $H_3$), ~ 4.9 (1H, —O*H* and —$CO_2$*H*), 5.1 (1H, d, J $\approx$ 5Hz, $H_5$); $\nu_{max}$ (film) 3100 (br), 1770, 1740, 1310, 1115 $cm^{-1}$; m/e 169 and 124 prominent.

## Example 5

Benzyl 6-$\beta$-benzylpenicillanate

A solution of benzyl 6-$\alpha$-benzyl-6-$\beta$-isocyanopenicillanate (prepared by the methods of Bentley and Clayton, J.C.S. Chem Comm, 1974 p 278) (0.220 g, 0.542 mmol) in dry benzene (7 $cm^3$) was treated with ABIBN (20 mol%) followed by tri-*n*-butyl tin hydride (0.181 g), 0.623 mmol) and the mixture heated under reflux (nitrogen atmosphere) for 12 hours. Following removal of the solvent under reduced pressure, column chromatography on silica-gel (20 g, 35% EtOAc-petroleum ether as eluant) gave benzyl 6-$\beta$-benzylpenicillanate (0.150 g, 72%), containing traces of alkyl-tin residues. Rechromatography (silica-gel) gave a pure sample of the penam as an oil, $[\alpha]_D^{20} = {}^+348.4°$ ($CHCl_3$); $\nu_{max}$ (film) 1770 (lactam C = O), 1740 $cm^{-1}$ (ester C = O); $\delta$ ($CDCl_3$) 1.38 and 1.65 (each 3H, s, *gem*-$Me_2$), 3.1 (2H, d, J —Hz, 6-$\beta$-C*$H_2$*), 3.86 (1H, dt, *J* 8, 4.4, $C_6$—*H*), 4.45 (1H, s, $C_3$—*H*), 5.16 (2H, s, PhC*$H_2$*—), 5.37 (1H, d, *J* 4.4Hz, $C_5$—*H* — collapses to singlet on irradiation of $C_6$—*H* multiplet), 7.23 and 7.35 (each 5H, s, Ph—*H*); m/e 381 ($M^+$), 91($C_7H_7^+$— parent ion); Accurate mass 381.1403 observed, $C_{22}H_{23}NO_3S$ requires 381.1397.

## Example 6

6-$\beta$-Benzylpenicillanic acid

Benzyl 6-$\beta$-benzylpenicillanate (96 mg, 0.25 mmol) was dissolved in ethyl acetate (5 ml) and treated with hydrogen on Pd-C catalyst (5%, 200 mg) at 30 psi overnight. Filtration and removal of solvent gave 82 mg of an oil, shown by nmr to be a mixture of the title compound and starting material. Three further treatments with hydrogen over fresh catalyst (10%, 70—100 mg), followed by extraction into base, acidification and reextraction into chloroform gave a low yield of the title compound; δ ($CDCl_3$) 1.56, 1.72 (6H, 2s, *gem*-$Me_2$), 3.12 (2H, d, J ≈ 7Hz, pH—$CH_2$—), ~3.8 (1H, m, $H_6$), 4.43 (1H, s, $H_3$), 5.4 (1H, d, J ≈ 4Hz, $H_5$), 7.24 (5H, s, $C_6H_5$—), 8.1 (1H, broad s, —$CO_2H$); $\nu_{max}$ (film) 3100 (br), 1770 (sh), 1740 $cm^{-1}$.

Example 7

Benzyl 6-β-benzyl-1,1-dioxopenicillanate

To a solution of benzyl 6-β-benzylpenicillanate (0.080 g, 0.21 mmol) in chloroform (3 $cm^3$) was added *m*-chloroperbenzoic acid (0.090 g, 0.525 mmol). After stirring the mixture at room temperature for 3 days the precipitated *m*-chlorobenzoic acid was removed by filtration, and the filtrate washed successively with 5% aqueous sodium sulphite (5 $cm^3$) and 5% aqueous sodium bicarbonate (5 $cm^3$). Evaporation of the dried ($MgSO_4$) organic layer under reduced pressure gave an oil (0.090 g, 100%) which after chromatography on silica gel (5 g, 30% EtOAc-petrol) afforded benzyl 6-β-benzyl-1,1-dioxopenicillanate (0.043 g, 50%); $\nu_{max}$ (film) 1795 (lactam C = O), 1750 (ester C = O), 1310 and 1120 $cm^{-1}$ (O = S = O); δ ($CDCl_3$) 1.22 and 1.53 (each 3H, s, *gem*-$Me_2$), 3.35 (2H, m, 6-β-$CH_2$), 4.1 (1H, m, $C_6$—*H*), 4.50 (1H, s, $C_3$—*H*), 4.5 (1H, d, *J* 4.7Hz, $C_5$—*H*), 5.21 (2H, ABq, $J_{gem}$ 11.7Hz, Ph$CH_2$O—), 7.28 and 7.36 (each 5H, s, Ph—*H*); m/e 413 ($M^+$), 349 ($M^+$ —$SO_2$), and 91 (parent ion, $C_7H_7^+$).

Example 8

6-β-Benzyl-1,1-dioxopenicillanic acid

Benzyl 6-β-benzyl-1,1-dioxopenicillanate (140 mg, 0.339 mmol) was dissolved in ethyl acetate (5 ml) and shaken overnight with hydrogen on 10% Pd-C catalyst (120 mg) at 55 psi. The suspension was then filtered and the filtrate evaporated to give the title compound (97 mg, 88%) as white crystals; δ ($CDCl_3$) 1.44, 1.62 (6H, 2s, *gem*—$Me_2$), ~3.15 (1H, dd, $J_{gem}$ = 14.5Hz, $J_{6,8}$ = 7Hz, $H_8$), ~3.55 (1H, dd, $J_{gem}$ = 14.5Hz, $J_{6,8}$ = 10Hz, $H_8'$), ~4.2 (1H, ddd, J = 10, 7, 5Hz, $H_6$), 4.52 (1H, s, $H_3$), ~4.55 (1H, d, J = 5Hz, $H_5$), 7.29 (5H, s, $C_6H_5$—), 8.09 (1H, broad s, —$CO_2H$); $C_{15}H_{17}NO_5S$ requires m/e 323.0825 measured m/e 323.0823; $\nu_{max}$ (film) 3200 (br), 1795, 1760, 1320 and 1115 $cm^{-1}$.

Example 9

Benzyl 6-β-methoxycarbonylethylpenicillanate

A solution of benzyl 6-β-isocyano-6-α-methoxycarbonylethylpenicillanate (prepared by the methods of Bentley and Clayton, J.C.S. Chem. Comm., 1974 p 278) (0.334 g, 0.831 mmol) in dry benzene (10 $cm^3$) was treated with ABIBN (0.027 g, 20 mol%) followed by tri-*n*-butyl tin hydride (0.253 mg, 0.873 mmol), and the mixture heated under reflux (nitrogen atmosphere). Following removal of solvent under reduced pressure, chromatography on silica gel (15 g, 20% EtOAc-petrol as eluant) gave benzyl 6-β-methoxycarbonylethylpenicillanate (0.193 g, 62%) contaminated with a trace of alkyl-tin compounds. Rechromatography (silica-gel) afforded a pure oil, $[\alpha]_D^{20}$ = + 160.1 ($CHCl_3$); $\nu_{max}$(film) 1770 (lactam C = O), 1735 $cm^{-1}$ (ester C = O); δ ($CDCl_3$) 1.39 and 1.58 (each 3H, s, *gem*-$Me_2$), 2.0—2.5 (4H, m, —$CH_2CH_2CO_2$—), 3.5—3.8 (1H, m, $C_6$—*H*), 3.64 (3H, s, —$CO_2$*Me*), 4.41 (1H, s, $C_3$—*H*), 5.16 (2H, s, Ph-$CH_2$), 5.41 (1H, d, *J* 4.4Hz, $C_5$—*H* — collapses to singlet or irradiation of $C_6$—H multiplet), 7.35 (5H, s, Ph—*H*; m/e 377 ($M^+$), 250, accurate mass 377.1295 observed, $C_{19}H_{23}NO_5S$ requires 377.1295.

Example 10

6-β-Methoxycarbonylethylpenicillanic acid

Benzyl 6-β-methoxycarbonylethylpenicillanate (100 mg, 0.265 mmol) was dissolved in ethyl acetate (5 ml) and treated with hydrogen at 55 psi on 10% Pd-C catalyst (100 mg) overnight. Filtration of the suspension through celite followed by evaporation of solvent gave unchanged ester 100 mg). This material was then hydrogenated over fresh catalyst for 2½ hrs, and showed some free acid on work-up (analysis by nmr). Three further treatments with hydrogen/fresh catalyst gave almost complete conversion to the free acid, which was purified by extraction into base, acidification and re-extraction to yield the title compound as a clear oil (42 mg, 53%); δ ($CDCl_3$) 1.57, 1.67 (6H, 2s, *gem*-$Me_2$), 2.0—2.5 (4H, m, —$CH_2CH_2CO_2$—), 3.6—3.7 (1H, m, $H_6$), 3.67 (3H, s, —$CO_2CH_3$), 4.39 (1H, s, $H_3$), 5.4 (1H, d, J = 4.4Hz, $H_5$), 8.1 (broad s, —$CO_2H$); $\nu_{max}$ 3200 (br), 1740 (sh) and 1730; m/e 287, 259, 187, 160, 128, 114 and 100.

Example 11

Benzyl 6-β-methoxycarbonylmethylpenicillanate

A solution of benzyl 6-isocyano-6-methoxycarbonylmethylpenicillanate (prepared by the methods of Bentley and Clayton, J.C.S. Chem. Comm. 1974 p 278) (0.257 g, 0.66 mmol) (mixture of isomers — predominantly β-isocyano) in dry benzene (7 $cm^3$) was treated with ABIBN (20 mol%) followed by tri-*n*-butyl tin hydride (0.231 g, 0.792 mmol), and the mixture heated under reflux (nitrogen atmosphere) for 10 min. Following removal of solvent under reduced pressure, column chromatography of the crude product on silica-gel (25% EtOAc-petrol as eluant) gave benzyl 6-β-methoxycarbonylmethyl-penicillanate (0.146 g, 61%), and subsequently (1R, 5R)-6-(1-benzyloxycarbonyl-2-methylprop-1-yl)-1-methoxycarbonylmethyl-4-thia-2,6-diazabicyclo (3.2.0)hept-2-en-7-one (0.015 g, 6%) both slightly contaminated with alkyltin residues. Rechromatography of the $\beta$-alkylpenicillanate gave a pure sample as a clear oil, $[\alpha]_D^{20} = +218.9°$ ($CHCl_3$); $\nu_{max}$ (film) 1775 (lactam C = O), 1735 $cm^{-1}$ (ester C = O), $\delta$ ($CDCl_3$) 1.18 and 1.24 (each 3H, s, *gem*-$Me_2$), 2.56 (1H, dd, $J_{gem}$ 17.6Hz, $J$ 5.5Hz, $C_8$—$H$), 2.80 (1H, dd, $J_{gem}$ 17.6Hz, $J$ 11Hz, $C_8$—$H'$), 3.25 (3H, s, —$CO_2CH_3$), 3.58 (1H, m, $J$ 5.5, 11, and 4.5Hz, $C_6$—$H$), 4.28 (2H, ABq, $J_{gem}$ 13.2Hz, $PhCH_2$—), 5.46 (1H, d, $J$ 4.5Hz, $C_5$—$H$), 7.18 (5H, s, Ph—$H$); m/e 363 ($M^+$), 91 (parent ion, $C_7H_7^+$); Accurate mass 363.1151 observed, $C_{18}H_{21}NO_5S$ requires 363.1139.

Example 12

Benzyl 6-$\beta$-t-butoxycarbonylmethylpenicillanate

i) *Benzyl 6-$\alpha$-t-butoxycarbonylmethyl -6-$\beta$-isocyanopenicillanate*

A solution of benzyl 6-isocyanopenicillanate (prepared by the methods of Bentley and Clayton J.C.S. Chem Comm., 1974 p 278) (1.39 g, 4.4 mmol) in dry DMF (20 $cm^3$) was treated with potassium carbonate (0.607 g, 4.4 mmol) and *t*-butyl bromoacetate (0.853 g, 4.4 mmol). The mixture was then stirred overnight at room temperature and worked up by pouring into ice-water (150 $cm^3$) followed by extraction with chloroform (3 x 50 $cm^3$). The organic layer was washed successively with water (2 x 50 $cm^3$) and brine (50 $cm^3$), dried over $MgSO_4$ and then the solvent removed under vacuum to give a crystalline product (1.71 g, 90%). Column chromatography on silica-gel (50 g, 35%) EtOAc-petrol) gave firstly benzyl 6-$\alpha$-t-butoxycarbonylmethyl-6-$\beta$-isocyanopenicillanate (0.940 g, 50%) as yellowish crystals, and then a by-product (1R, 5R)-6-(1-benzyloxycarbonyl-2-propen-1-yl)-1-butoxycarbonylmethyl-4-thia-2,6-diazabicyclo(3.2.0)hept-2en-7-one (0.275 g, 14.5%) as a golden oil. Recrystallisation of a sample of the penicillanate yielded colourless crystals, m.p. 153—4°C; $\nu_{max}$($CCl_4$) 2120 (N = C), 1795 (lactam C = O), 1740 $cm^{-1}$ (ester C = O); $\delta$ ($CDCl_3$) 1.42 and 1.64 (each 3H, s, *gem*-$Me_2$), 1.47 (9H, s, $Bu^t$-$H$), 3.02 (2H, ABq, $J_{gem}$ 16Hz, 6-$\alpha$-$CH_2$), 4.55 (1H, s, $C_3$—$H$), 5.19 (2H, s, $PhCH_2$—), 5.50 (1H, s, $C_5$—$H$), 7.37 (5H, s, Ph—$H$); m/e 430 ($M^+$), 91 (parent ion, $C_7H_7^+$); Accurate mass 430.1575 observed, $C_{22}H_{26}N_2O_5S$ requires 430.1562. (Found C, 61.25; H, 6.11; N, 6.37; $C_{22}H_{26}N_2O_5S$ requires C, 61.37; H, 6.09; N, 6.51).

Rechromatography (silica-gel) of the thiazoline by-product gave an oil; $\nu_{max}$ (film) 1770 (lactam C = O), 1710 $cm^{-1}$ $\delta$ ($CDCl_3$) 1.37 (9H, s, $Bu^t$—$H$), 1.90 and 2.26 (each 3H, s, *gem*—$Me_2$), 3.0 (2H, ABq, $J_{gem}$ 15Hz, 6-$\alpha$-$CH_2$), 5.18 (2H, s, $PhCH_2$—), 5.75 (1H, d, $J$ 2Hz, $C_5$—$H$), 7.36 (5H, s, Ph—$H$), 7.93 (1H, d, $J$ 2Hz, $C_3$—$H$); m/e 430 ($M^+$), 91 (parent ion, $C_7H_7^+$).

ii) *Benzyl 6-$\beta$-t-butoxycarbonylmethylpenicillanate*

A solution of benzyl 6-$\alpha$-*t*-butoxycarbonylmethyl-6-$\beta$-isocyanopenicillanate (0.181 g, 0.42 mmol) in dry benzene (5 $cm^3$) was treated with ABIBN (20 mol%) followed by tri-*n*-butyl tin hydride (0.135 g, 0.462 mmol), and the mixture heated under reflux (nitrogen atmosphere) for 60 min. Removal of solvent under reduced pressure was followed by silica-gel chromatography (10 g, 25% EtOAc-petrol as eluant) which gave benzyl 6-$\beta$-t-butoxycarbonylmethylpenicillanate (0.09 g, 53%), slightly contaminated with alkyl-tin residues; $\nu_{max}$ (film) 1780 (lactam C = O), 1750 sh, 1730 (ester C = O's); $\delta$ ($CDCl_3$) 1.40 and 1.68 (each 3H, s, *gem*-$Me_2$), 1.42 (9H, s, $Bu^t$-$H$), 2.7 (2H, m, 6- $\beta$-$CH_2$), 3.7—4.1 (1H, octet, $J$ 4, 7.5, 9 Hz, $C_6$—$H$), 4.43 (1H, s, $C_3$—$H$), 5.18 (2H, s, $PhCH_2$—), 5.54 (1H, d, $J$ 4Hz, $C_5$—$H$), 7.35 (5H, s, Ph—$H$); m/e 405 ($M^+$), 91 (parent ion, $C_7H_7^+$). This material has also been prepared by the methods of Sheehan et al., J. Org. Chem., 1977 p 4045.

Example 13

Benzyl 6-$\beta$-carboxymethylpenicillanate

Ice-cold trifluoroacetic acid (1 $cm^3$) was used to dissolve benzyl 6-$\beta$-*t*-butoxycarbonylmethyl penicillanate (0.075 g, 0.185 mmol), and the solution stirred for 30 min. at 0°C. Trifluoroacetic acid was then removed under reduced pressure, and the remaining oil freeze-dried from benzene to give benzyl 6-$\beta$-carboxymethylpenicillanate as an oil which solidified on storage. Precipitation from EtOAc-petrol gave a white, amorphous solid (52 mg, c. 50%), $\nu_{max}$(film) 3000br(—OH), 1740(lactam C = O), 1720sh, 1670 $cm^{-1}$ (ester and acid C = O's); $\delta$ ($CDCl_3$) 1.41 and 1.54 (each 3H, s, *gem*-$Me_2$), 2.98 (2H, m, 6-$\beta CH_2$), 3.36 (1H, m, $C_6$—$H$), 4.46 (1H, s, $C_3$—$H$), 5.20 (2H, s, $PhCH_2$), 5.73 (1H, d, $J$ 6.45Hz, $C_5$—$H$), 7.36 (5H, s, Ph—$H$), 8.77 (2H, broad s, —$CO_2H$ = $H_2O$ of hydration). m/e 349($M^+$), 91 (parent ion, $C_7H_7$); Accurate mass 349.0981 observed, $C_{17}H_{19}NO_5S$ requires 349.0983. This material has also been prepared by the methods of Sheehan et al., J. Org. Chem. 1977, p 4045.

Example 14

Benzyl 6$\beta$-methylpenicillanate

i) *Benzyl 6$\beta$-formamido-6$\alpha$-methylpenicillanate*

Benzyl 6$\beta$-amino-6$\alpha$-methylpenicillanate, $\delta$ 1.41 (3H, s, $CH_3$), 1.51 br (6H, s, $CH_3$), 1.94 (2H, s, $NH_2$), 4.43 (1H, s, 3—H), 5.19 (2H, s, $CO_2CH_2$), 5.23 (1H, s, 5—H), 7.37 (5H, s, Ph—$H$) was prepared from the toluene-p-sulphonate salt (2.49 g, 3.1 mmol). The dry amine was then dissolved in pyridine (15 ml), cooled to −40°C prior to the dropwise addition of formic-acetic anhydride (6 ml), then allowed to reach room temperature over an hour. The solvent was removed under vacuum and the residue

taken up in chloroform before washing with water, dilute HCl, aqueous sodium bicarbonate and brine. Removal of solvent gave the title compound as a pale yellow oil which crystallised on standing (1.56 g, 90%); m.p. 104—105°C (from EtOAc-light petroleum); $[\alpha]_D^{20}$ 282° (c 0.64); $\nu_{max}$ 3 300, 1 780, 1 740, 1 680, 1 525 cm$^{-1}$; $\delta$ 1.38, 1.55, 1.83 (9H, 3s, C$H_3$), 4.42 (1H, s, 3—H), 5.19 (2H, s, $CO_2CH_2$), 5.40 (1H, s, 5—H), 6.55 br (1H, s, N*H*), 7.37 (5H, s, Ph—*H*), 8.15 (1H, d, J = 1.2Hz, C*H*O) (Found: M$^+$ 348.1143. $C_{17}H_{20}N_2O_4S$, M requires 348.1142).

ii) *Benzyl 6β-isocyano-6α-methylpenicillanate*

Benzyl 6β-formamido-6α-methylpenicillanate (0.75 g, 2.15 mmol) was dissolved in dry dichloromethane (10 ml) and cooled before addition of triethylamine (0.6 ml, 4.32 mmol). The solution was further cooled under nitrogen to −70°C, and a solution of phosgene (0.213 g, 2.15 mmol) in dichloromethane added slowly with stirring. As the solution warmed to room temperature a rapid precipitation of triethylamine hydrochloride occurred; this precipitate was filtered off and the filtrate washed with brine before drying and removal of solvent. The pale yellow oil thus obtained crystallised on standing to give the title compound (0.54 g, 76%), m.p. 74—76°C (from EtOAc-light petroleum); $[\alpha]_D^{20}$ 137° (c 0.60); $\nu_{max}$ 2 130, 1 790 and 1 740 cm$^{-1}$; $\delta$ 1.41, 1.62, 1.84 (9H, 3s, C$H_3$); 4.54 (1H, s, 3—H), 5.19 (2H, s, $CO_2CH_2$), 5.24 (1H, s, 5—H), 7.37 (5H, s, Ph—*H*). (Found: M$^+$ 330.103 8. $C_{17}H_{18}N_2O_3S$, M requires 330.103 8).

iii) *Benzyl 6β-methylpenicillanate*

Benzyl 6β-isocyano-6α-methylpenicillanate (0.3 g, 0.91 mmol) was dissolved in dry benzene (6 ml), then ABIBN (trace) and tri-*n*-butyl tin hydride (0.277 g, 5% excess) added. The solution was then heated to reflux under nitrogen for 15 minutes, before removal of solvent at reduced pressure. An attempt was made to free the same of alkyl-tin residues by partitioning between acetonitrile and hexane;* this was partially successful, but some penam material was lost, and chromatography was still necessary. Thus the title compound was obtained as an oil, (0.1 g, 36%); $[\alpha]_D^{20}$ 159° (c 0.42); $\nu_{max}$ 1 770, 1 740 cm$^{-1}$; $\delta$ 1.29 (3H, d, *J* 7.6Hz, 6β-C$H_3$), 1.40 and 1.60 (6 H, 2s, C$H_3$), 3.6 (1H, dd, *J* 4.5 and 7.6Hz, 6—H), 4.41 (1H, s, 3—H), 5.18 (2H, s, $CO_2CH_2$), 5.43 (1H, d, *J* 4.5Hz, 5—H), 7.36 (5H, s, Ph—*H*). (Found M$^+$ 305.1087. $C_{16}N_{19}NO_3S$, M requires 305.1091).

## Example 15

Sodium 6β-methylpenicillanate

Benzyl 6β-methylpenicillanate (0.05 g, 0.164 mmol) was dissolved in ethyl acetate (3 ml) and hydrogenated at one atmosphere pressure over 10% Pd/C catalyst (0.05 g) for one hour. Catalyst and solvent were then renewed and the process repeated; after five such changes the remaining material (0.02 g) was shown by pmr to comprise approximately 80% free acid and 20% benzyl ester. The mixture was then dissolved in dry acetone (0.2 ml) and a solution of sodium ethyl hexanoate in MIBK (2 molar, 39 μl) added with shaking. Precipitation of the title *salt* was aided by dilution with dry ether (2 ml), and ether was used to wash the collected solid (0.013 4 g, 34%). $\nu_{max}$(KBr disc) 1 765 and 1 750 (lactam C = O**), 1 605 and 1 410 cm$^{-1}$; $\delta$ ($D_2O$, 80 MHz) (HOD at 4.70) 1.16 (3H, d, *J* 8Hz, 6β-C$H_3$), 1.42 and 1.54 (6H, 2s, C$H_3$), 3.65 (1H, dd, *J* 4 and 8Hz, 6—H), 4.06 (1H, s, 3—H), 5.37 (1H, d, *J* 4Hz, 5—H).

## Example 16

Benzyl 1,1-dioxo-6β-methylpenicillanate

Benzyl 6β-methylpenicillanate (0.055 g, 0.18 mmol) was treated with *m*-chloroperbenzoic acid (0.093 g, 50% excess) in chloroform (3 ml) overnight. Chromatography afforded the title compound as an oil, (0.045 g, 74%), $[\alpha]_D^{20}$ 142° (c 1.73); $\nu_{max}$ 1 795, 1 750, 1 320 and 1 115 cm$^{-1}$; $\delta$ 1.26 and 1.51 (6H, s, C$H_3$), 1.55 (3H, d, *J* 7.8Hz, 6—C$H_3$), 3.85 (1H, dd, *J* 5 and 7.8Hz, 6—H), 4.46 (1H, s, 3—H), 4.55 (1H, d, *J* 5Hz, 5—H), 5.22 (2H, ABq, *J* 12Hz, $CO_2CH_2$), 7.37 (5H, s, Ph—*H*).

## Example 17

Sodium 1,1-dioxo-6β-methylpenicillanate

Benzyl 1,1-dioxo-6β-methylpenicillanate (0.04 g) was dissolved in ethyl acetate (4 ml) and hydrogenated over 10% Pd/C for 30 minutes. Removal of catalyst left the *free acid* of the title compound as a glass (0.025 g, 85%), $[\alpha]_D^{20}$ 189° (c 0.38); $\nu_{max}$ 1 795, 1 720, 1 320 and 1 115 cm$^{-1}$. This was then converted to its *sodium salt*, as a white, granular solid (0.017 g, 78%), $\nu_{max}$ (KBr disc) 1 770, 1 605, 1 405 and 1 119 cm$^{-1}$; $\delta$ ($D_2O$, 80MHz) (HOD at 4.70) 1.37 and 1.49 (6H, 2s, C$H_3$), 1.41 (3H, d, *J* 6.1Hz, 6—C$H_3$), ca. 4.0 (1H, m, 6—H) 4.16 (1H, s, 3—H), 4.90 (1H, d, *J* 5.1Hz, 5—H).

---

* J.M. Berge and S.M. Roberts, Synthesis 471 (1979).

** Doubling of band apparently due to a lattice effect.

## Example 18

Benzyl penicillanate

A solution of benzyl 6-β-bromopenicillanate (See Example 29) (0.113 g, 0.306 mmol) was treated with ABIBN (20 mol%) and tri-*n*-butyl tin hydride (0.100 g, 0.344 mmol), then heated under reflux (nitrogen atmosphere) for 15 min. The solvent was then removed under reduced pressure and the residue purified by silica-gel chromatography (10 g, 25% EtOAc-petrol as eluant) to yield benzyl penicillanate (0.068 g, 75%) as an oil, $\nu_{max}$ (film) 1780 (lactam C = O), 1745 $cm^{-1}$ (ester C =O); δ ($CDCl_3$) 1.38 and 1.61 (each 3H, s, *gem*-$Me_2$), 3.0 (1H, dd, $J_{gem}$ 15Hz, $J$ 2.0Hz, $C_6$—*H*), 3.55 (1H, dd, $J_{gem}$ 15Hz, $J$ 4.5Hz, $C_6$—*H*), 4.48 (1H, s, $C_3$—*H*), 5.16 (2H, s, $PhCH_2$), 5.25 (1H, dd, $J + J = 6.5$Hz, $C_5$—*H*), 7.33 (5H, s, Ph—*H*).

## Example 19

Benzyl 6-β-methylthiopenicillanate

To a solution of benzyl 6-β-isocyano-6-α-methylthiopenicillanate (prepared by the methods of Bentley and Clayton J.C.S. Chem. Comm., 1974, p 278) (0.075 g, 0.207 mmol) in dry benzene (5 $cm^3$) were added ABIBN (20 mol%) and tri-*n*-butyl tin hydride (0.064 g, 0.217 mmol), and the mixture heated under reflux (nitrogen atmosphere) for 60 min. The solvent was then removed under reduced pressure and silica-gel chromatography (3.5 g, 20% EtOAc-petrol) of the crude product afforded benzyl 6-β-methylthiopenicillanate as an oil, $\nu_{max}$ (film) 1780 (lactam C = O), 1740 $cm^{-1}$ (ester C = O); δ ($CDCl_3$) 1.40 and 1.64 (each 3H, s, *gem*-$Me_2$), 2.28 (3H, s, —$SCH_3$), 4.4 (1H, d, $J$ 4.5Hz, $C_6$—*H*) 4.48 (1H, s, $C_3$—*H*), 5.18 (2H, s, $PhCH_2$—), 5.53 (1H, d, $J$ 4.5Hz, $C_5$—*H*), 7.34 (5H, s, Ph—*H*).

## Example 20

2,2,2-Trichloroethyl 6β-chloropenicillanate

i) *2,2,2-Trichloroethyl 6β-phenylacetamidopenicillanate*

The potassium salt of benzylpenicillin (44.52 g, 0.119M) was suspended in 600 ml of dry methylene chloride. A slurry of pyridinium chloride (14.40 g, 0.119M) in 60 ml $CH_2Cl_2$ was added. A solution of trichloroethanol (18.0 g, 0.119M) in 60 ml of $CH_2Cl_2$ and then dicyclohexylcarbodiimide (24.72 g, 0.119M) in 120 ml $CH_2Cl_2$ were added. The mixture was stirred for 17 hrs at room temperature and then filtered. The filtrate was washed with 5% sodium bicarbonate (3 x 200 ml), water (1 x 200 ml), dried ($MgSO_4$) and the solvent removed under reduced pressure. The resultant crude yellow/brown solid was recrystallised from ethyl acetate/petroleum ether (60—80) to give the ester as white crystals 38.90 g (70%): m.p. 157—158°; I.R. ($CHCl_3$): 3 300, 1 770 and 1 690 $cm^{-1}$; NMR ($CDCl_3$) δ 1.49 (S, 6H) 3.55 (S, 3H) 4.45 (S, 1H) 4.76 (S, 2H) 5.50 (m, 2H) 6.30 (broad d, 1H) 7.20 (S, 5H).

ii) *2,2,2-Trichloroethyl 6β*-N-Nitrosophenylacetamidopenicillanate

Dinitrogen tetroxide (20 g, 0.217M) was dissolved in 250 ml methylene chloride. Half of this was then added to a mixture of 2,2,2-trichloroethyl 6β-phenylacetamidopenicillanate (31.50 g, 0.067 mM) and sodium acetate (66 g, 0.80 M) in 350 ml $CH_2Cl_2$ and the mixture stirred for 1½ hrs at −5°. The remaining dinitrogen tetroxide was added after 45 mins. Excess dinitrogen tetroxide was destroyed by pouring onto sodium bicarbonate solution (60 g in 500 ml $H_2O$) slowly, with stirring over a period of approx 30 mins. The organic phase was washed with aq. sodium bicarbonate (1 x 500 ml) water (1 x 500 ml), dried ($MgSO_4$) and concentrated to approx. 350 ml. This is the N-nitrosophenylacetamidopenicillanate, which was not isolated but converted immediately to the diazo derivative.

iii) *2,2,2-Trichloroethyl 6-diazopenicillanate*

2,2,2-Trichloroethyl 6β-N-nitrosophenylacetamidopenicillanate was refluxed in methylene chloride for 4 hrs, allowed to cool, washed with aqueous sodium bicarbonate solution (3 x 250 ml), water (1 x 500 ml), dried ($MgSO_4$) and the solvent removed under reduced pressure to give the crude yellow solid diazo compound. Recrystallisation from ethanol gave the 2,2,2-trichloroethyl 6-diazopenicillanate as yellow crystals 12.42 g (51%): m.p. 103—104°; IR ($CHCl_3$) 2100 and 1740 $cm^{-1}$; NMR δ 1.50 (S, 3H) 1.70 (S, 3H) 4.45 (S, 1H) 4.70 (S, 2H) 6.15 (S, 1H).

iv) *2,2,2-Trichloroethyl 6-chloro-6-phenylselenylpenicillanate*

2,2,2-Trichloroethyl 6-diazopenicillanate (1.5 g, 4.19 mM) was dissolved in 50 ml $CH_2Cl_2$ under a nitrogen atmosphere. Phenylselenyl chloride (0.802 g, 4.19 mM) in 25 ml $CH_2Cl_2$ was added, slowly, with stirring over 5—10 mins. There was an immediate evolution of nitrogen and simultaneous decolouration of the phenyl selenyl chloride. The mixture was then stirred for 15 min at room temperature, and the solvent removed under reduced pressure to give 1.7 g crude yellow/brown oil which slowly solidified. Recrystallisation from ethylacetate/petroleum ether (60—80) gave 2,2,2-trichloroethyl 6-chloro-6-phenylselenylpenicillanate as white crystals 1.6 g (53%): m.p. 115—115.5°; IR ($CHCl_3$) 1780 and 1760 $cm^{-1}$, NMR ($CDCl_3$) δ 1.58 (S, 3H) 1.83 (S, 3H) 4.69 (S, 1H) 4.79 (S, 2H) 5.68 (S, 1H) 7.37 (m, 4H) 7.79 (m, 2H). Anal. Calculated for $C_{16}H_{15}NO_3Cl_4$ S se (522.20): C, 36.80; H, 2.89; N, 2.68; Cl, 27.16; S, 6.14. Found: C, 36.98; H, 3.03; N, 2.59; Cl, 26.88; s, 6.00: $[\alpha]_D^{20} = +89.83°$.

v) *2,2,2-Trichloroethyl 6β-chloropenicillanate*

2,2,2-Trichloroethyl 6-chloro-6-phenylselenylpenicillanate (500 mg, 0.95 mM) was dissolved in dry benzene (about 25 ml). ABIBN (30 mg, 20 mol%) was then added, followed by tri-n-butyl tin hydride (290 mg, 1.10 mol). The solution was then heated to a gentle reflux for 1 hr under a nitrogen atmosphere (oil bath at 88°). TLC of the crude mixture showed two spots, and separation of the two by column chromatography on silica gel (15 g) in 5% ethyl acetate/petroleum ether (60—80) gave starting material (104 mg) and the required 6β-chloro-penicillanate as a pale yellow oil (166 mg, 44%): IR ($CHCl_3$); 1785 and 1760 $cm^{-1}$; NMR ($CHCl_3$) δ 1.60 (S, 3H) 1.70 (S, 3H) 4.46 (S, 1H) 4.80 (S, 2H) 5.25 (d, 1H, J = 4Hz) 5.65 (d, 1H, J = 4Hz); $[\alpha]_D^{20} = +125°$.

Complete reaction could not be achived by increasing either the reaction time or the amount of ABIBN or $nBu_3SnH$.

Example 21

6β-Chloropenicillanic acid

2,2,2-Trichloroethyl 6β-chloropenicillanate (100 mg, 0.27 mM) was dissolved in 90% acetic acid (5 ml). The solution was then cooled to 0° before zinc dust (acid washed, 1.0 g) was added. The mixture was then stirred at 0° for 4 hrs. The zinc was removed by filtration under vacuum into a flask containing 100 ml of ice water and washing of the zinc with 50 ml methylene chloride yielded a two phase system. The organic layer was separated off and the aqueous layer washed three time with methylene chloride (3 x 50 ml). The combined organic phases were dried ($MgSO_4$) and evaporated under reduced pressure (no heat) to give the free acid as a pale yellow oil.

For purification, the acid was dissolved in methylene chloride (50 ml) and extracted with aqueous sodium bicarbonate. The aqueous layer, after being extracted several times with methylene chloride (3 x 75 ml) was cooled in ice and acidified with dilute HCl (pH 1 → 2). Extraction with methylene chloride, drying ($MgSO_4$) and removal of solvent under reduced pressure (no heat) afforded the pure acid, as an off-white solid 38 mg (60%). $[\alpha]_D$ + 256° (0.2% in $CHCl_3$), $\nu_{max}$($CHCl_3$) 2600 (OH), 1790 (β-lactam C=O), 1730 (acid C=O), 1300 $cm^{-1}$; δ ($CDCl_3$) 1.58 and 1.69, (each 3H, S, gem-$Me_2$), 4.54 (1H, S, $NCHCO_2H$), 5.23 (1H, d, *J* 3.8Hz, ClC*H*CHS), 5.60 (1H, d, *J* 4.1Hz, ClCHC*H*S) 7.26 br (1H, S, $CO_2H$) [addition of $D_2O$ causes the signal at 7.26 to disappear]; m/e 237, 235 ($M^+$), 160 ($M^+ - ClC{=}\overset{+}{C}{=}O$) and 100 (100%).

Example 22

Preparation of pivaloyloxymethyl 6β-bromopenicillanate (method 1)

6α/β-Bromopenicillanic acid (10 g) was dissolved/suspended in dimethylformamide (600 ml) containing methylene chloride (200 ml) and was treated sequentially with triethylamine (12 ml) and bromomethyl pivalate (10.4 ml) with stirring at 0°C. The reaction was allowed to warm to room temperature and was monitored by thin layer chromatography. After 2 hours, the reaction mixture was evaporated in vacuo to approx. 100 ml; it was diluted with chloroform (400 ml), washed with water (2 x 400 ml), dried ($MgSO_4$), and evaporated in vacuo to afford a gum. This gum was subjected to column chromatography on silica gel (80 g) using ethyl acetate:cyclohexane (1:5 → 1:7). The appropriate fractions were combined and evaporated in vacuo to afford pivaloyloxymethyl 6β-bromopenicillanate as an impure gum. This was resubjected to column chromatography on silica gel (80 g) using ethyl acetate:cyclohexane (1:8 → 1:10) as eluant). The appropriate fractions were combined and evaporated in vacuo to afford pivaloyloxymethyl 6β-bromopenicillanate (0.23 g), i.r. (liq. film) 1790, 1750 $cm^{-1}$, n.m.r. ($CDCl_3$) 1.20 (9H, s, $C(CH_3)_3$), 1.48 (3H, s, $CH_3$), 4.52 (1H, s, *H*-3), 5.32 and 5.56 (2H, 2d, *J* = 4Hz, *H*-5 and *H*-6), 5.8 (2H, ABq *J* = 5Hz, —$CH_2$—) ppm.

(Combination and evaporation in vacuo of some earlier fractions afforded a mixture of pivaloyloxymethyl 6α-bromopenicillanate and the title compound (1:1) (0.25 g) which could be rechromatographed if desired).

Example 23

Preparation of pivaloyloxymethyl 6β-bromopenicillanate (method 2a)

*i) Pivaloyloxymethyl 6,6-dibromopenicillanate*

To 6,6-dibromopenicillanic acid (prepared by the methods of Clayton, J.C.S. (c) 1969, p 2123) (5 g) in dry N,N-dimethylformamide (50 ml) was added solid potassium carbonate (1.9 g) followed by bromomethyl pivalate (2.6 ml). The mixture was stirred at room temperature for 1 h and then poured into ice-water (200 ml). Extraction with ethyl acetate (2 x 150 ml) gave an oil which was chromatographed over silica gel (150 g). Elution with light petrol-ethyl acetate gave pivaloyloxymethyl 6,6-dibromopenicillanate (4.5 g), m.p. 101—103°C.

*ii) Pivaloyloxymethyl 6β-bromopenicillanate*

Pivaloyloxymethyl 6,6-dibromopenicillanate (1.5 g) and α,α[1]-azoisobutyronitrile (0.11 g) in benzene (40 ml) was added tri-n-butyltin hydride (0.88 ml) and the stirred mixture was kept at 90° under reflux in a nitrogen atmosphere for 1 h. The cooled reaction mixture was then evaporated to dryness and the residue partitioned between n-hexane and acetonitrile. The acetonitrile extract was

evaporated to dryness and the residue was chromatographed over silica gel (50 g). Elution of the column with light petrol-ethyl acetate (9:1) gave pivaloyloxymethyl 6β-bromopenicillanate (0.28 g), $[\alpha]_D^{20}$ + 158.9 (*c*, 1.1; $CHCl_3$); $\nu_{max}$ ($CHCl_3$) 1790, 1770 and 1750 cm$^{-1}$; $\delta_H$($CDCl_3$) 5.78 (2H, dd, *J* = 8.5Hz, *J* = 7Hz, —$OCH_2O$—), 5.54 (1H, d, *J* = 4Hz, 5-C*H*), 5.30 (1H, d, *J* = 4Hz, 6-C*H*), 4.50 (1H, s, 3-C*H*), 1.65 (3H, s, 2-$CCH_3$), 1.48 (3H, s, 2-$CCH_3$), and 1.20 (9H, s, —$C(CH_3)_3$) (Found: $M^+$ 393.0217. $C_{14}H_{20}NO_5SBr$ requires $M^+$ 393.02).

## Example 24

Preparation of pivaloyloxymethyl 6β-bromopenicillanate (method 2b)

*i) Pivaloyloxymethyl 6,6-dibromopenicillanate*

6,6-Dibromopenicillanic acid (1 g, 2.78 mmol) was dissolved in dry dichloromethane (8 ml) by the addition of triethylamine (0.31 g, 3.07 mmol). The solution was chilled in an ice-salt bath and pivaloyloxymethyl bromide (0.6 g, 10% excess) added in dichloromethane (2 ml). The reaction was then allowed to reach room temperature, and stirred for two hours before the addition of water (20 ml). The organic phase was washed with aqueous sodium bicarbonate and brine before drying ($MgSO_4$) and removal of solvent to leave an oil (1.2 g, 90%).

Chromatography afforded the ester as a crystalline solid (0.98 g, 68%), m.p. 109—110°C (ether-light petroleum); $[\alpha]_D^{20}$ + 171° (c 0.13); $\nu_{max}$ (KBr disc) 1795, 1775 and 1755 (both ester C=O)**; δ 1.20 (9H, s, $C(CH_3)_3$), 1.47 and 1.60 (6H, 2s, $CH_3$), 4.54 (1H, s, 3-H), 5.76 (1H, s, 5-H), 5.82 (2H, ABq, *J*Hz, $CO_2CH_2C_2C$), (Found M+ 470.936 8. $C_{14}H_{19}NO_5SBr_2$, M requires 470.938 6).

*ii) Pivaloyloxymethyl 6β*-bromopenicillanate

Pivaloyloxymethyl 6,6-dibromopenicillanate (2.1 g, 4.37 mmol) was dissolved in dry benzene (15 ml), then ABIBN (trace) and triphenyl tin hydride (1.53 g, 4.37 mmol) added. The solution was then refluxed under nitrogen for 10 minutes before removal of solvent at reduced pressure. Examination of the reaction mixture by pmr showed a product composition of 23:18:49:10 $Br_2$:α-Br:β-Br:$H_2$ respectively. A similar treatment of the *Benzyl ester* with tri-*n*-butyl tin hydride has given a product ratio of 28:7; 40:25.

The crude product, dissolved in chloroform, was then stirred with aqueous potassium fluoride.* A white precipitate formed immediately, and following filtration, removal of solvent gave a mixture of penams (2 g) essentially free of alkyl-tin residues. Chromatography afforded a mixture of starting material and the *6α-bromopenam* (0.45 g), the *6β-bromopenam* (0.6 g, 35%) and the *dihydropenam* (0.15 g, 11%). The sample of pivaloyloxymethyl 6β-bromopenicillanate crystallised on removal of solvent.

## Example 25

Preparation of pivaloyloxymethyl 6β-bromopenicillanate (method 3)

Methyllithium (*ca* 2M, 4 ml) was added to a solution of pivaloyloxymethyl 6,6-bromopenicillanate (2.75 g) in toluene (150 ml) at −78° under nitrogen. The mixture was stirred at −78° for 10 min. Acetic acid (1 ml) in toluene (10 ml) was then added and the mixture was allowed to come to room temperature. This was washed with water, dried and evaporated to dryness. The residue was chromatographed over silica gel (60 g). Elution of the column with light petrol-ethyl acetate (4:1) gave pivaloyloxymethyl 6β-bromopenicillanate (0.2 g) identical to an authentic sample (t.l.c. and n.m.r. comparisons).

## Example 26

Preparation of phthalidyl 6β-bromopenicillanate

*i) Phthalidyl 6,6-dibromopenicillanate*

Potassium carbonate (1.0 g) followed by bromophthalide (3.0 g) was added to a solution of 6,6-dibromopenicillanic acid (5.0 g) in N,N-dimethylformamide (30 ml) and the mixture was stirred at room temperature overnight. This was then poured into ice-water (200 ml). Extraction with ethyl acetate (2 × 150 ml) gave an oil which was chromatographed over silica gel (100 g). Elution with light petrol-ethyl acetate gave phthalidyl 6,6-dibromopenicillanate (2.5 g) as a glass. $\nu_{max}$(KBr) 1795, 1285, 1170 and 980 cm$^{-1}$; $\delta_H$ ($CDCl_3$) 7.50—8.05 (4H, m, Ar*H*), 7.41, 7.45

(1H, 2s, —OC*H*—O),

5.69, 5.79 (1H, 2s, 5-C*H*), 4.58, 4.60 (1H, 2s, 3-C*H*), 1.60 (3H, s, 2-$CCH_3$), and 1.43, 1.45 (3H, 2s, 2-$CCH_3$).

---

* J.E. Liebner and J. Jacobus, J. Org. Chem. *44*, 449. (1978).

** Band doubling apparently due to a lattice effect.

*ii) Phthalidyl 6β-bromopenicillanate*

Tri-n-butyltin hydride (0.65 g) was added to a solution of phthalidyl 6,6-dibromopenicillanate (1 g) and $\alpha,\alpha^1$-azoisobutyronitrile (0.07 g) in benzene (40 ml) and the mixture was boiled under reflux under nitrogen for 1 h. The cooled reaction mixture was then evaporated to dryness and the residue partitioned between n-hexane and acetonitrile. The acetonitrile extract was evaporated to dryness and the residue was chromatographed over silica gel (40 g). Elution with light petrol-ethyl acetate (1:1) afforded phthalidyl 6β-bromopenicillanate (0.35 g) as a foam, $\nu_{max}(CHCl_3)$ 1790 and 980 cm$^{-1}$; $\delta_H$ ($CDCl_3$) 7.50—8.05 (4H, m, Ar*H*), 7.43, 7.48

(1H, 2s, —OC*H*—O),

5.50, 5.57 (1H, 2d, $J = 4.5$Hz, 5-C*H*), 5.39, 5.43 (1H, 2d, $J = 4.5$Hz, 6-C*H*), 4.46, 4.48 (1H, s, 3-C*H*), 1.63 (3H, m, 2-C$CH_3$), and 1.55 (3H, s, 2-C$CH_3$) (Found: $M^+$ 410.9740. $C_{16}H_{14}NO_5SBr$ requires $M^+$ 410.9774).

Example 27

Preparation of N-phthalimidomethyl 6,6-dibromopenicillanate

*i) N-Phthalimidomethyl 6,6-dibromopenicillanate*

To a solution of N-(hydroxymethyl)phthalimide (11 g) in T.H.F. (150 ml) at −10° was added $SOCl_2$ (4.5 ml) in T.H.F. and dry $Et_3N$ (8.6 ml) with stirring. The mixture was allowed to warm slowly to room temperature, with continuous stirring, and then kept at room temperature for 0.5 h before an equal volume of dry benzene was added. The precipitated triethylamine hydrochloride was filtered. Evaporation of the filtrate yielded the N-(chloromethyl)-phthalimide (11.5 g) as an off white solid.

N-(Chloromethyl)phthalimide (7.7 g) was dissolved in dry D.M.F. (50 ml) containing $K_2CO_3$ (0.9 g) with continuous stirring. The solution was stirred at room temperature for a further 2 h and then poured onto ice-water which was extracted with ethyl acetate (3 x 70 ml). The ethyl acetate extract was dried and evaporated to yield a red-brown oil, which after silica gel column chromatography (50% EtOAc in petrol as eluant) yielded N-phthalimidomethyl 6,6-dibromopenicillanate as a colourless oil $[\alpha]_D$ + 80.0° (*c*, 1.4; $CHCl_3$); $\nu_{max}(CHCl_3)$ 1790, 1750, and 1740 cm$^{-1}$; $\delta_H$ ($CDCl_3$) 7.90 (4H, m, Ar*H*), 5.81 (3H, br s, 5-C*H* and —O$CH_2$N—), 4.52 (1H, s, 3-C*H*), 1.60 (3H, s, 2-C$CH_3$), and 1.49 (3H, s, 2-C$CH_3$).

*ii) N-Phthalimidomethyl 6β-bromopenicillanate*

To a solution of N-phthalimidomethyl 6,6-dibromopenicillanate (2.5 g) in dry benzene (50 ml) was added $\alpha,\alpha^1$-azoisobutyronitrile (0.16 g) and tri-n-butyltin hydride (15 ml). The mixture was refluxed under nitrogen for 1 h, after which the benzene was evaporated to yield a red-brown oil. This was dissolved in acetonitrile (100 ml) and extracted with n-hexane (5 x 100 ml). The acetonitrile was evaporated under reduced pressure to yield a pale yellow oil which after silica-gel column chromatography (20% EtOAc in petrol as eluant) yielded N-phthalimidomethyl 6β-bromopenicillanate as needles, m.p. 95—97° (from light petrol-ethyl acetate, $[\alpha]_D$ + 141.2° (*c*, 1.1; $CHCl_3$), $\nu$ ($CHCl_3$) 1795, 1750 and 1740 cm$^{-1}$; $\delta$ ($CDCl_3$) 7.85 (4H, m, Ar*H*), 5.79 (2H, s, —O$CH_2$N—), 5.85 (1H, d, $J = 4$Hz, 5-C*H*), 5.30 (1H, d, $J = 4$Hz, 6-C*H*), 4.52 (1H, s, 3-C*H*), 1.62 (3H, s, 2-C$CH_3$), and 1.50 (3H, s, 2-C$CH_3$).

Example 28

Sodium 6β-bromopenicillanate

Sodium 6,6-bromopenicillanate (4.5 g) in water (100 ml) was layered with ethyl acetate (100 ml). Aqueous hydrochloric acid (5 M) was added to the stirred mixture dropwise until pH 1.5 was reached. The organic layer was separated, washed with brine, dried, and evaporated to 30 ml. N,O-Bistrimethylsilylacetamide (3.5 ml) was added and the mixture was stirred at room temperature for 0.5 h. The mixture was then evaporated to dryness at 50° and 0.05 torr. The residue (4.67 g) was dissolved in dry benzene (50 ml). $\alpha,\alpha^1$-Azoisobutyronitrile (0.4 g) and tri-n-butyltin hydride (3 ml) were added to the stirred solution which was then boiled under reflux under nitrogen for 1 h. The cooled reaction mixture was evaporated to dryness and the residue partitioned between n-hexane and acetonitrile. The acetonitrile extract was added water (2 ml) and methanol (2 ml) followed by sodium ethyl-hexanoate in methyl isobutyl ketone (2M, 5.5 ml). The mixture was then evaporated to dryness. Addition of diethyl ether (200 ml) to the residue gave a white precipitate which was filtered and washed with diethyl ether (400 ml) to give a mixture (3.02 g) containing sodium 6β-bromopenicillanate (50%), sodium 6α-bromopenicillanate (12%), sodium 6,6-dibromopenicillanate (12%), and sodium penicillanate (26%).

Example 29

Benzyl 6-β-bromopenicillanate

A solution of benzyl 6,6-dibromopenicillanate (prepared by the method of DiNinno et al., J. Org. Chem, 1977, *42*, 2960) (0.293 g, 0.652 mmol) in dry benzene (7 cm$^3$) was treated with ABIBN (20 mol %) followed by tri-*n*-butyl tin hydride (0.189 g, 0.652 mmol) and the mixture heated under reflux (nitrogen atmosphere) for 10 min. Removal of the solvent under reduced pressure led to an oily residue

which consisted of four major components (other than alkyl-tin compounds); resulting from varying degrees of reduction. Chromatography of this product mixture on silica-gel (35 g, 25% EtOAc-petrol as eluant) led to the identification (tlc and nmr spectroscopy of the following fractions; in order of elution: the starting 6,6-dibromopenicillanate (0.040 g); a mixture of benzyl 6-$\alpha$-bromo- (30%) and 6-$\beta$-bromopenicillanate (70%) (0.100 g); benzyl 6-$\beta$-bromopenicillanate (0.050 g), and benzyl penicillanate (0.054 g). All fractions were slightly contaminated with alkyl-tin residues. Rechromatography (silica-gel) of the 6-$\beta$-compound afforded a pure sample of benzyl 6-$\beta$-bromopenicillanate as an oil, $\nu_{max}$ (film) 1785 (lactam C=O), 1740 cm$^{-1}$ (ester C=O); $\delta$ ($CDCl_3$) 1.40 and 1.63 (each 3H, s, *gem*-$Me_2$), 4.54 (1H, s, $C_3$-*H*), 5.18 (2H, s, $PhCH_2$), 5.2 (1H, d, *J* 4.4Hz, $C_6$-*H*), 5.55 (1H, d, *J* 4.4Hz; $C_5$-*H*), 7.36 (5H, s, Ph-*H*); m/e 369 ($M^+$), 91 (parent ion; $C_7H_7^+$); Accurate Mass 369.0044 observed $C_{15}H_{16}NO_3BrS$ requires 369.0033.

Example 30

Trichloroethyl 6$\beta$-bromopenicillanate

*i) Trichloroethyl 6,6-dibromopenicillanate*

6,6-Dibromopenicillanic acid (1.52 g, 4.25 mmol) was dissolved in dry dichloromethane (30 ml) by addition of pyridine (0.336 g, 1 eq), the dicyclohexylcarbodi-imide (0.877 g, 1 eq) and tri-chloroethanol (0.635 g, 1 eq) in dichloromethane (30 ml total) were added. The mixture was stirred vigorously overnight before removal of the precipitate by filtration and evaporation of the filtrate to a brown gum. This was then taken up in ether (50 ml) and refiltered before washing with 1N HCl (30 ml), $H_2O$ (30 ml), $NaHCO_3$ (5%, 30 ml) and drying over $MgSO_4$. Evaporation gave a pale brown oil (2.09 g, > 100%) which on chromatography (60 g silica gel, 20% EtOAc-petrol) yielded a clear oil (1.5 g, 81%); $\delta$ ($CDCl_3$) 1.54, 1.65 (6H, 2s, *gem*-$Me_2$), 4.67 (1H, s, $H_3$), 4.88 (2H, s, $—CO_2CH_2—$) 5.83 (1H, s, $H_5$); $\nu_{max}$ 1800 and 1760 cm$^{-1}$; m/e 487, 312, 216, 198 and 114.

Trichloroethyl 6,6-dibromopenicillanate (alternative preparation)

Trichloroethyl 6-diazopenicillanate (0.5 g, 1.4 mmol was dissolved in dichloromethane (10 ml) and cooled to below —45°C under nitrogen. Bromine (0.22 g, 1.4 mmol) in dichloromethane (10 ml) was then added over 30 mins, and the stirred solution allowed to warm to room temperature. The solvent was removed under vacuum to give a dark oil, yielding the title compound as an oil (430 mg, 63%) after chromatography.

*ii) Trichloroethyl 6-$\beta$-bromopenicillanate*

Trichloroethyl 6,6-dibromopenicillanate (175 mg, 0.38 mmol) was dissolved in dry benzene (5 ml) then ABIBN (20 mol %) and tri-*n*-butyltin hydride (111 mg, 1 eq) were added, before refluxing under nitrogen for 1 hr. Chromatography on silica gel gave a sample of the title compound as an oil (67 mg, 43%); $\delta$ ($CDCl_3$) 1.56, 1.71 (6H, 2s, *gem*-$Me_2$), 4.56 (1H, s, $H_3$), 4.79 (2H, s, $—CO_2CH_2—$), 5.35 (1H, d, J = 4Hz, $H_6$), 5.61 (1H, d, J = 4Hz, $H_5$); together with the 6$\alpha$-bromo and 6,6-dihydro compounds.

Example 31

Methoxymethyl 6$\beta$-bromopenicillanate

*i) Methoxymethyl 6,6-dibromopenicillanate*

6,6-Dibromopenicillanic acid (2 g, 5.59 mmol) was dissolved in dry dichloromethane by the addition of triethylamine (0.78 ml, 5.6 mmol) and cooled to —78°C. A solution of chloromethyl methyl ether* (1.95 ml, 11.18 mmol) in dry dichloromethane (4 ml) was then added and the solution allowed to warm to room temperature before evaporating to dryness in vacuo. The residue was dissolved in chloroform (25 ml) then washed with water (2 x 20 ml) and $NaHCO_3$ (10 ml, 5%) before drying over $MgSO_4$. Evaporation gave a buff crystalline solid (2.09 g, 93%), recrystallised from ethyl acetate-petrol to give the title compound as 1.84 g (81%) pale yellow needles; $\delta$ ($CDCl_3$) 1.52, 1.64 (6H, 2s; *gem*-$Me_2$), 3.49 (3H, s; $—OCH_3$), 4.57 (1H, s; $H_3$), 5.33 (2H, q = 5.9Hz; $—CO_2CH_2—$), 5.81 (1H, s; $H_5$); $\nu_{max}$ 1795 and 1745 cm$^{-1}$; m/e 403, 324, 314, 257, 204, 198, 144, 114 and 45.

*ii) Methoxy 6-$\beta$-bromopenicillanate*

Methoxymethyl 6,6-dibromopenicillanate (500 mg, 1.24 mmol) was dissolved in dry benzene (7 ml) then ABIBN (20 mol %) and tri-*n*-butyl tin hydride (369 mg, 1.24 mmol) were added. The solution was refluxed for 30 mins before removal of solvent in vacuo. NMR spectroscopy of the crude product mixture indicated in presence of starting material, 6-$\alpha$-bromo, 6-$\beta$-bromo, and 6,6-dihydro penams.

---

*Potent carcinogen — all operations carried out in fume cupboard.

Chromatography (25 g silica gel, 30% EtOAc-petrol) yielded the title compound as a clear oil (140 mg, 36%); $\delta$ ($CDCl_3$) 1.51, 1.68 (6H, 2s, *gem*-$Me_2$), 3.46 (3H, s, $OCH_3$), 4.50 (1H, s, $H_3$), 5.27 (2H, s, —$CO_2CH_2O$—), 5.37 (1H, d, J = 4.5 Hz, $H_6$), 5.59 (1H, s, J = 4.5 Hz, $H_6$); $\delta$ ($Me_2CO$-$d_6$) 1.26, 1.41 (6H, 2s, *gem*-$Me_2$), 3.08 (3H, s, —$OCH_3$), 4.48 (1H, s, $H_3$), 4.70 (1H, d, J = 4 Hz, $H_5$), 4.97 (2H, g, $J_{gem}$ = 6 Hz, —$CO_2CH_2O$—), 5.16 (1H, d, J = 4 Hz, $H_5$); $\nu_{max}$ 1785 and 1750 $cm^{-1}$; m/e 323, 295, 244, 234, 204, 179, 144, 114 and 45.

Example 32

6$\beta$-Bromopenicillanic acid (method 1)

Methoxymethyl 6$\beta$-bromopenicillanate (155 mg, 0.5 mmol) was dissolved in ice-cold trifluoroacetic acid (3 ml) and stirred at 0°C for 45 minutes. The solution was then concentrated (at 0°C) under vacuum, before freeze-drying twice from benzene (2 × 4 ml) to afford the title compound as a granular orange residue (37 mg); $\delta$ ($CD_3NO_2$) 1.35, 1.58 (2s, *gem-$Me_2$*), 4.7 (s, $H_3$), 5.2 (d, J = 5.5Hz, $H_6$), 5.9 (d, J = 5.5Hz, $H_5$) ppm. This material proved to be rather unstable.

Sodium 6$\beta$-bromopenicillanate (method 2)

Methoxymethyl 6$\beta$-bromopenicillanate (0.16 g) in trifluoroacetic acid (3 ml) was stirred at 0° for 10 min. The mixture was evaporated to dryness at 0°. The residue was azeotroped with toluene (2 × 50 ml) at 0° to give an off-white powder which was dissolved in acetonitrile (5 ml). Sodium ethylhexanoate in methyl isobutyl ketone (2*M*, 0.23 ml) was then added and the mixture diluted with diethyl ether (100 ml). The precipitate was then filtered, and washed with diethyl ether (100 ml) to give sodium 6$\beta$-bromopenicillanate as a microcrystalline solid (0.13 g), $[\alpha]_D^{20}$ + 185° (*c*, 0.8; $H_2O$), $\nu_{max}$ (KBr) 1775, 1758, 1610, 1405, 1318, 1228, 1123, 782 and 632 $cm^{-1}$, $\delta_H$ (DMSO-$d_6$) 5.66 (1H, d, *J* = 4Hz, 5-C*H*), 5.51 (1H, d, *J* = 4Hz, 6-C*H*), 4.00 (1H, s, 3-C*H*), 1.52 (3H, s, 2-$CCH_3$) and 1.46 (3H, s, 2-$CCH_3$).

Example 33

Benzyl 6$\beta$-bromo-1,1-dioxopenicillanate

*i) Benzyl 6,6-dibromo-1,1-dioxopenicillanate*

To a solution of benzyl 6,6-dibromopenicillanate (0.320 g, 0.71 mmol) in chloroform (5 $cm^3$) was added *m*-chloroperbenzoic acid (0.307 g, 1.78 mmol). After stirring the mixture at room temperature for 4 days the precipitated *m*-chlorobenzoic acid was removed by filtration and the filtrate washed successively with 10% aqueous sodium sulphite (10 $cm^3$) and 10% aqueous sodium bicarbonate (10 $cm^3$). Evaporation of the dried ($MgSO_4$) organic layer under reduced pressure afforded benzyl 6,6-dibromo-1,1-dioxopenicillanate (0.295 g, 86%) as a crystalline solid, m.p. 145—147°C (from EtOAc-petrol), $[\alpha]_D^{20}$ = +154.8° ($CHCl_3$), $\nu_{max}$ (film) 1810 (lactam C=O), 1755 $cm^{-1}$ (ester C=O); $\delta$ ($CDCl_3$) 1.26, 1.55 (each 3H, s, *gem*-$Me_2$), 4.54 (1H, s, $C_3$—H), 5.00 (1H, s, $C_5$—*H*), 5.60 (2H, q, Ph—$CH_2$), 7.37 (5H, s, Ph—*H*), m/e 479 ($M^+$), 415 ($M^+$—$SO_2$) and 91 ($C_7H_7^+$). (Found: C, 37.19; H, 3.13; N, 2.90; Br, 32.84; S, 6.74. $C_{15}H_{15}NO_5BrS$ requires C, 37.44; H, 3.14; N, 2.91; Br, 33.21; S, 6.66%).

*ii) Benzyl 6-$\beta$-bromo-1,1-dioxopenicillanate*

A solution of benzyl 6,6-dibromo-1,1-dioxopenicillanate (0.089 g, 0.186 mmol) in dry benzene (5 $cm^3$) was treated with tri-*n*-butyltin hydride (0.064 g, 0.22 mmol) and the mixture refluxed under nitrogen for 50 min. Removal of the solvent under vacuum led to an oily residue which on fractionation by silica-gel chromatography (EtOAc-petrol, 3:7 as eluant) afforded three major fractions. The first eluted material (0.023 g) was identical (tlc and nmr spectroscopy) to a mixture of starting material and benzyl 6-$\alpha$-bromo-1,1-dioxopenicillanate. The intermediate fraction proved to be the required benzyl 6-$\beta$-bromo-1,1-dioxopenicillanate (0.030 g, 40%) whilst the finally eluted material was identified (tlc and nmr spectroscopy) as benzyl 1,1-dioxopenicillanate (0.0125 g, 21%). Re-chromatography of the 6-$\beta$-bromo-compound (on silica-gel) in order to remove the last traces of alkyl-tin residues afforded the title compound as an oil (0.018 g), $\nu_{max}$ (film) 1810 (lactam C=O), 1755 (ester C=O), 1335 and 1120 $cm^{-1}$ (O=S=O); ($CDCl_3$) 1.25 and 1.55 (each 3H, s, *gem*-$Me_2$), 4.53 (1H, s, $C_3$—*H*), 4.85 (1H, d, *J* 4.5Hz, $C_6$—H), 7.35 (5H, s, Ph—*H*).

Example 34

*(i) Pivaloyloxymethyl 6,6-dibromo-1,1-dioxopenicillanate*

Pivaloyloxymethyl 6,6-dibromopenicillanate (0.5 g, 1.05 mmol) was treated overnight with *m*-chloroperbenzoic acid (0.36 g, 2.1 mmol) in chloroform (10 ml). The resulting suspension was filtered to remove spent per-acid, and the filtrate washed with aqueous sodium bicarbonate and brine, then dried before evaporation of solvent to give an oil. Chromatography afforded the title compound (0.2 g, 38%), m.p. 115—117°C (ether — light petroleum); $[\alpha]_D^{20}$ 161° (c 0.5); $\nu_{max}$ 1810, 1755, 1340, 1110 and 965 $cm^{-1}$; $\delta$ 1.20 (9H, s, $C(CH_3)_3$), 1.42 and 1.68 (6H, 2s, $CH_3$), 4.52 (1H, s, 3-*H*), 5.00 (1H, s, 5-H), 5.74 and 5.94 (2H, ABq, *J* 6Hz, $CO_2CH_2O_2C$).

*(ii) Pivaloyloxymethyl 6$\beta$-bromo-1,1-dioxopenicillinate*

Treatment of pivaloyloxymethyl 6,6-dibromo-1,1-dioxopenicillanate (0.13 g, 0.257 mmol) with tri-*n*-butyl tin hydride (0.75 g, 0.257 mmol) and ABIBN (trace) in refluxing benzene (4 ml) for ten

minutes under nitrogen gave, following repeated chromatography, a pure sample of the title compound, as an oil $[\alpha]_D^{20}$ 127.9° (c 0.53); $\nu_{max}$ 1810, 1770 sh, 1750, 1330, 1110 and 970 $cm^{-1}$; $\delta$ 1.23 (9H, s, $C(CH_3)_3$), 1.42 and 1.60 (6H, 2s, $CH_3$), 4.53 (1H, s, 3-H), 4.78 (1H, d, $J$ 4.5Hz, 5-H), 5.35 (1H, d, $J$ 4.5Hz 6-H) 5.71 and 5.95 (2H, ABq, $J$ 5 Hz, $CO_2CH_2O_2C$).

## Example 35

Benzyl 6$\beta$-chloropenicillanate

Benzyl 6-chloro-6-phenylselenylpenicillanate (900 mg, 1.9 mM) was dissolved in anhydrous benzene (20 ml). ABIBN (62 mg, 0.37 MM) was added, followed by tri-n-butyltin hydride (607 mg, 2.08 mM). The solution was then heated under reflux under a nitrogen atmosphere for 1.5 h. Evaporation of solvent in vacuo gave a colourless oil which showed a single component in addition to the tri-n-butyltinphenylselenium residues. Purification of the crude product by column chromatography on silica-gel [ethyl acetate — light petroleum (1:19) as eluant] gave, as a colourless oil, benzyl 6$\beta$-chloropenicillanate (448 mg, 74%), $[\alpha]_D$ + 249° (1% in $CHCl_3$), $\nu_{max}$ ($CHCl_3$) 1790 ($\beta$-lactam C=O), 1740 (ester C=O), 1500, 1460, 1030, 760 (C—Cl) $cm^{-1}$; $\delta$ ($CDCl_3$) 1.35 and 1.55 (each 3H, S, *gem*-$Me_2$), 4.50 (1H, S, $NCHCO_2CH_2Ph$), 5.15 (2H, S, $CO_2CH_2Ph$), 5.17 (1H, d, $J$ 4Hz, ClCHCHS), 5.55 (1H, d, $J$ 4Hz, ClCHCHS), 7.30 (5H, S, $CO_2CH_2Ph$); m/e 326, 324, ($M^+$), 251, 249 ($M^+$—$Cl\overset{+}{C}$=C=O), and 91 (100%, $C_7H_7^+$) (Found: $M^+$, 325.0513. $C_{15}H_{15}$ $^{35}ClNO_3S$ requires M, 325.0530.

## Example 36

2,2,2-Trichloroethyl 6$\beta$-chloropenicillanate 1,1-dioxide

2,2,2-Trichloroethyl 6$\beta$-chloropenicillanate (168 mg, 0.48 mM) was dissolved in anhydrous dichloromethane (10 ml). m-Chloroperoxybenzoic acid (1.97 mg, 1.14 mM) was added, and the reaction mixture stirred at room temperature for 24h. The reaction mixture was then diluted with dichloromethane (20 ml), washed with aqueous sodium bicarbonate, then water, dried ($MgSO_4$), and evaporated in vacuo to give a white solid (119 mg, 80%). Recrystallisation from ethyl acetate — light petroleum, gave white crystals of 2,2,2-trichloroethyl 6$\beta$-chloropenicillanate 1,1-dioxide (95 mg, 64%), m.p. 157—158°C, $[\alpha]_D$ + 157° (0.7% in $CHCl_3$), $\nu_{max}$ ($CHCl_3$) 1820 ($\beta$-lactam C=O), 1770 (ester C=O), 1340 ($SO_2$), 1180, 810 $cm^{-1}$, $\delta$ ($CDCl_3$) 1.50 and 1.70 (each 3H, s, *gem*-$Me_2$), 4.71 (1H, d, $J$ = 4Hz, ClCHCHS), 4.85 (2H, q, $J$ 4.7 and 19.6Hz, $CO_2CH_2CCl_3$), 4.96 (1H, S, $NCHCO_2CH_2CCl_3$), 5.39 (1H, d, $J$ 4.1Hz, ClCHCHS); m/e 401, 399, 397, ($M^+$—Cl), and 100 (100%) (Found: C, 30.11, H, 2.93; Cl, 35.29; N, 3.32; S, 8.18. $C_{10}H_{11}$$^{35}Cl_4NO_5S$ requires C, 30.09; H, 2.78; Cl, 35.54; N, 3.51; S, 8.03%).

## Example 37

Preparation of Benzyl 6$\beta$-chloropenicillanate 1,1-dioxide

Benzyl 6$\beta$-chloropenicillanate (300 mg, 0.92 mM) was dissolved in anhydrous dichloromethane (15 ml). m-Chloroperoxybenzoic acid (397 mg, 2.3 mM) was added, and the reaction mixture stirred for 24h at room temperature. Work-up as in example 36, followed by column chromatography on silica-gel (ethyl acetate — light petroleum (1:9) as eluant) gave, as a colourless oil, benzyl 6$\beta$-chloropenicillanate 1,1-dioxide (316 mg, 96%) $[\alpha]_D$ + 193° (1% in $CHCl_3$), $\nu_{max}$ ($CHCl_3$) 1810 ($\beta$-lactam C=O), 1745 (ester C=O), 1460, 1340 ($SO_2$), 1120 ($SO_2$), 700 $cm^{-1}$; $\delta$ ($CDCl_3$) 1.25 and 1.54 (each 3H, S, gem-$Me_2$), 4.56 (1H, S, $NCHCO_2CH_2Ph$), 4.77 (1H, d, $J$ 4.7Hz, ClCHCHS), 5.23 (2H, q, $J$ 4.4 and 9.0Hz, $CO_2CH_2Ph$), 5.33 (1H, d, $J$ 4.7Hz, ClCHCHS), 7.38 (5H, S, $CO_2CH_2Ph$); m/e 358, 356 ($M^+$) and 91 (100%, $C_7H_7^+$).

## Example 38

6$\beta$-Chloropenicillanic acid 1,1-dioxide

Benzyl 6$\beta$-chloropenicillanate acid 1,1-dioxide (100 mg, 0.28 mM) was dissolved in ethyl acetate and 10% Pd/C catalyst (100 mg) added to it. The mixture was then placed in an atmosphere of hydrogen at 55 p.s.i. for 1.5h. The catalyst was removed by filtration, and the solvent evaporated in vacuo to yield, as a white foam, 6$\beta$-chloropenicillanic acid 1,1-dioxide (60 mg, 80%), $\nu_{max}$ (nujol) 2600, 2400 (OH), 1810 ($\beta$-lactam C=O), 1340 ($SO_2$), 1120 ($SO_2$), 1000, 840 $cm^{-1}$; $\delta$ [$(CD_3)_2C$=O)] 1.48 and 1.60 (each 3H, S, gem-$Me_2$), 4.54 (1H, S, $CHCO_2H$), 5.26 (1H, d, $J$ 4.4Hz, ClCHCHS), 5.83 (1H, d, $J$ 4.11Hz, ClCHCHS), 6.8 br (1H, S, OH) [addition of $D_2O$ causes the signal at 6.8 to disappear]; m/e 269, 267 ($M^+$) and 100 (100%).

**Claims for the Contracting States: BE CH IT NL SE**

1. A compound of the formula (I):

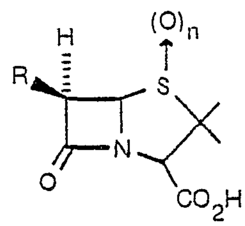

(I)

or a pharmaceutically acceptable salt or ester thereof wherein R is $C_{1-6}$ alkylthio, $C_{1-6}$ alkyl or $C_{1-6}$ alkyl substituted by phenyl, carboxy, $C_{1-6}$ alkoxycarbonyl, hydroxy or $C_{1-6}$ alkylthio, and n is one or two, with the proviso that R is not $C_{1-6}$ alkyl when n is one.

2. A compound as claimed in claim 1 wherein R is methylthio or ethylthio.

3. A compound as claimed in claim 1 wherein R is methyl, ethyl, n-propyl, isopropyl, n-butyl, sec-butyl or isobutyl.

4. A compound as claimed in claim 1 wherein R is benzyl, phenethyl or phenylpropyl.

5. A compound as claimed in claim 1 wherein R is carboxymethyl, carboxyethyl, carboxypropyl, methoxycarbonylmethyl, methoxycarbonylethyl, ethoxycarbonylmethyl or ethoxycarbonylethyl.

6. A compound as claimed in claim 1 wherein R is hydroxymethyl, 1-hydroxyethyl, 1-hydroxy-n-propyl, 2-hydroxy-n-propyl, 3-hydroxy-n-propyl, 1-hydroxyisopropyl or 2-hydroxyisopropyl.

7. A compound as claimed in claim 1 wherein R is methylthiomethyl or methylthioethyl.

8. A compound as claimed in any of claims 1 to 7 wherein n is two.

9. A compound of the formula (I):

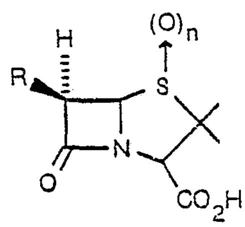

(I)

or a pharmaceutically acceptable salt or in-vivo hydrolysable ester thereof wherein R is fluoro, bromo, chloro or iodo, and n is zero, one or two with the proviso that when R is bromo or chloro and n is zero, the compound is contaminated by not more than 10% w/w of the corresponding 6$\alpha$- compound.

10. A compound as claimed in claim 9 wherein R is iodo.

11. A compound as claimed in claim 9 wherein R is bromo.

12. A compound as claimed in claim 9 wherein R is chloro.

13. A compound as claimed in any of claims 9 to 12 wherein n is zero.

14. A compound as claimed in any of claims 1 to 13 in the form of an alkali metal or alkaline earth metal salt.

15. A compound as claimed in any of claims 1 to 14 in the form of a sodium or potassium salt.

16. A compound as claimed in any of claims 1 to 13 in the form of an in-vivo hydrolysable ester.

17. A compound as claimed in any of claims 1 to 13 or 16 wherein the in-vivo hydrolysable ester group is acetoxymethyl, pivaloyloxymethyl, $\alpha$-acetoxyethyl, $\alpha$-acetoxybenzyl, $\alpha$-pivaloyloxyethyl, ethoxycarbonyloxymethyl, $\alpha$-ethoxycarbonylethyl, phthalidyl, 5,6-dimethoxyphthalidyl, dimethylaminomethyl, dimethylaminoethyl, diethylaminomethyl, diethylaminoethyl, N-phthalimidomethyl or methoxymethyl.

18. A compound in claim 1 which is sodium 1,1-dioxo-6$\beta$-methylpenicillanate.

19. A compound as claimed in claim 9 which is pivaloyloxymethyl 6$\beta$-bromopenicillanate when contaminated by not more than 10% of the corresponding 6$\alpha$- compound.

20. A compound as claimed in claim 9 which is sodium 6$\beta$-bromopenicillanate when contaminated by not more than 10% of the corresponding 6$\alpha$- compound.

21. A compound as claimed in claim 9 which is crystalline sodium 6$\beta$-bromopenicillanate.

22. A pharmaceutical composition which comprises penicillin or cephalosporin, a pharmaceutically acceptable carrier and a compound of the formula (I):

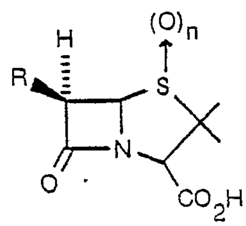

(I)

or a pharmaceutically acceptable salt or in-vivo hydrolysable ester thereof wherein R is halogen, $C_{1-6}$ alkylthio, $C_{1-6}$ alkyl or $C_{1-6}$ alkyl substituted by phenyl, carboxy, $C_{1-6}$ alkoxycarbonyl, hydroxy or $C_{1-6}$ alkylthio, and n is zero, one or two, with the proviso that when R is bromo and n is zero, the compound is contaminated by not more than 10% w/w of the corresponding 6$\alpha$- compound.

23. A pharmaceutical composition which comprises a compound as claimed in any of claims 1 to 21 and a pharmaceutically acceptable carrier.

24. A pharmaceutical composition which comprises a compound as claimed in any of claims 1 to 21, a penicillin or cephalosporin and a pharmaceutically acceptable carrier.

25. A compound as claimed in any of claims 1 to 21 for use in the treatment of bacterial infection.

26. A process for the preparation of a penicillin derivative of the formula (I):

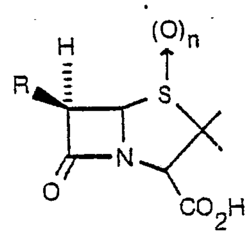

(I)

or a pharmaceutically acceptable salt or ester thereof, wherein R represents hydrogen, halogen, $C_{1-6}$ alkylthio, $C_{1-6}$ alkyl or alkyl substituted with phenyl, carboxy, $C_{1-6}$ alkoxycarbonyl, hydroxy or $C_{1-6}$ alkylthio, and n is zero, 1 or 2; which process comprises treating a compound of the formula (V):

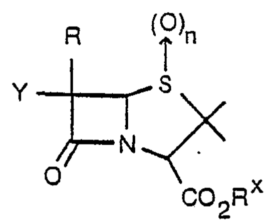

(V)

wherein either (a) R is as defined with respect to formula (I) and Y represents isocyano or halogen or (b) R is chloro and Y is arylselenyl; $R^x$ represents hydrogen or a carboxyl-blocking group, and n is zero 1 or 2; with a triaryl tin hydride, dialkyl tin dihydride or trialkyl tin hydride and thereafter optionally carrying out one or more of the following steps:

(i) converting a sulphide (n = 0), sulphoxide (n = 1), or sulphone (n = 2) to a different such group;
(ii) removing the carboxyl-blocking group if present;
(iii) converting the free penicillanic acid into a pharmaceutically acceptable salt or ester thereof.

27. A process as claimed in claim 26 wherein the reducing agent is a trialkyl tin hydride.

28. A process as claimed in claim 27 wherein the reducing agent is tri-*n*-butyl tin hydride.

29. A process as claimed in claim 26 wherein the reducing agent is triphenyl tin hydride.

30. A process for the preparation of an in-vivo hydrolysable ester of 6-$\beta$-bromopenicillanic acid when substantially free from the corresponding in-vivo hydrolysable ester of 6-$\alpha$-bromopenicillanic acid which process comprises preparing a mixture of isomeric in-vivo hydrolysable esters of 6$\alpha$- and 6$\beta$-bromopenicillanic acids and thereafter chromatographically separating the mixture of isomers into fractions and recovering the in-vivo hydrolysable ester of 6-$\beta$-bromopenicillanic acid from a fraction containing said in-vivo hydrolysable ester of 6-$\beta$-bromopenicillanic acid substantially free from the in-vivo hydrolysable ester of a 6-$\alpha$-bromopenicillanic acid.

**Claims for the Contracting States: DE FR GB**

1. A compound of the formula (I):

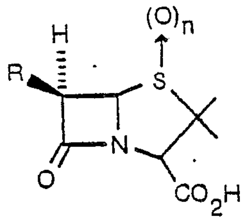

(I)

or a pharmaceutically acceptable salt or ester thereof wherein R is $C_{1-6}$ alkylthio, $C_{1-6}$ alkyl or $C_{1-6}$ alkyl substituted by phenyl, carboxy, $C_{1-6}$ alkoxycarbonyl, hydroxy or $C_{1-6}$ alkylthio, and n is one or two, with the proviso that R is not $C_{1-6}$ alkyl when n is one.

2. A compound as claimed in claim 1 wherein R is methylthio or ethylthio.

3. A compound as claimed in claim 1 wherein R is methyl, ethyl, n-propyl, isopropyl, n-butyl, sec-butyl or isobutyl.

4. A compound as claimed in claim 1 wherein R is benzyl, phenethyl or phenylpropyl.

5. A compound as claimed in claim 1 wherein R is carboxymethyl, carboxyethyl, carboxypropyl, methoxycarbonylmethyl, methoxycarbonylethyl, ethoxycarbonylmethyl or ethoxycarbonylethyl.

6. A compound as claimed in claim 1 wherein R is hydroxymethyl, 1-hydroxyethyl, 1-hydroxy-n-propyl, 2-hydroxy-n-propyl, 3-hydroxy-n-propyl, 1-hydroxyisopropyl or 2-hydroxyisopropyl.

7. A compound as claimed in claim 1 wherein R is methylthiomethyl or methylthioethyl.

8. A compound as claimed in any of claims 1 to 7 wherein n is two.

9. A compound of the formula (I):

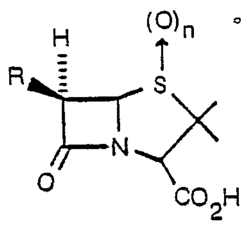

(I)

or a pharmaceutically acceptable salt or in-vivo hydrolysable ester thereof wherein R is fluoro, bromo, chloro or iodo, and n is zero, one or two with the proviso that when R is bromo and n is zero or two or R is chloro and n is zero then, in each case, the compound is contaminated by not more than 10% w/w of the corresponding 6$\alpha$- compound.

10. A compound as claimed in claim 9 wherein R is iodo.

11. A compound as claimed in claim 9 wherein R is bromo.

12. A compound as claimed in claim 9 wherein R is chloro.

13. A compound as claimed in any of claims 9 to 12 wherein n is zero.

14. A compound as claimed in any of claims 1 to 13 in the form of an alkali metal or alkaline earth metal salt.

15. A compound as claimed in any of claims 1 to 14 in the form of a sodium or potassium salt.

16. A compound as claimed in any of claims 1 to 13 in the form of an in-vivo hydrolysable ester.

17. A compound as claimed in any of claims 1 to 13 or 16 wherein the in-vivo hydrolysable ester group is acetoxymethyl, pivaloyloxymethyl, $\alpha$-acetoxyethyl, $\alpha$-acetoxybenzyl, $\alpha$-pivaloyloxyethyl, ethoxycarbonyloxymethyl, $\alpha$-ethoxycarbonylethyl, phthalidyl, 5,6-dimethoxyphthalidyl, dimethylaminomethyl, dimethylaminoethyl, diethylaminomethyl, diethylaminoethyl, N-phthalimidomethyl or methoxymethyl.

18. A compound as claimed in claim 1 which is sodium 1,1-dioxo-6$\beta$-methylpenicillanate.

19. A compound as claimed in claim 9 which is pivaloyloxymethyl 6$\beta$-bromopenicillanate when contaminated by not more than 10% of the corresponding 6$\alpha$- compound.

20. A compound as claimed in claim 9 which is sodium 6$\beta$-bromopenicillanate when contaminated by not more than 10% of the corresponding 6$\alpha$- compound.

21. A compound as claimed in claim 9 which is crystalline sodium 6$\beta$-bromopenicillanate.

22. A pharmaceutical composition which comprises a penicillin or cephalosporin, a pharmaceutically acceptable carrier and a compound of the formula (I):

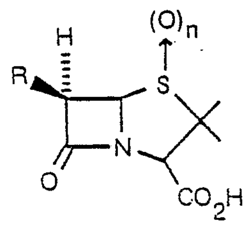

(I)

or a pharmaceutically acceptable salt or in-vivo hydrolysable ester thereof wherein R is halogen, $C_{1-6}$ alkylthio, $C_{1-6}$ alkyl or $C_{1-6}$ alkyl substituted by phenyl, carboxy, $C_{1-6}$ alkoxycarbonyl, hydroxy or $C_{1-6}$ alkylthio, and n is zero, one or two, with the proviso that when R is bromo and n is zero or two and the compound is contaminated by not more than 10% w/w of the corresponding 6$\alpha$- compound.

23. A pharmaceutical composition which comprises a compound as claimed in any of claims 1 to 21 and a pharmaceutically acceptable carrier.

24. A pharmaceutical composition which comprises a compound as claimed in any of claims 1 to 21, a penicillin or cephalosporin and a pharmaceutically acceptable carrier.

25. A compound as claimed in any of claims 1 to 21 for use in the treatment of bacterial infection.

26. A process for the preparation of a penicillin derivative of the formula (I):

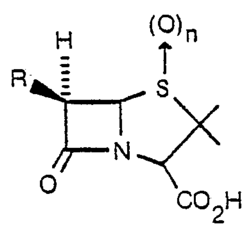

(I)

or a pharmaceutically acceptable salt or ester thereof, wherein R represents hydrogen, halogen, $C_{1-6}$ alkylthio, $C_{1-6}$ alkyl or alkyl substituted with phenyl, carboxy, $C_{1-6}$ alkoxycarbonyl, hydroxy or $C_{1-6}$ alkylthio, and n is zero, 1 or 2; which process comprises treating a compound of the formula (V):

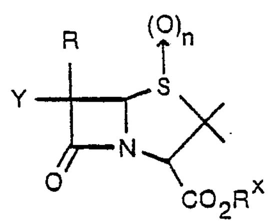

(V)

wherein either (a) R is as defined with respect to formula (I) and Y represents isocyano or halogen or (b) R is chloro and Y is arylselenyl; $R^x$ represents hydrogen or a carboxyl-blocking group, and n is zero 1 or 2; with a triaryl tin hydride, dialkyl tin dihydride or trialkyl tin hydride and thereafter optionally carrying out one or more of the following steps:

(i) converting a sulphide (n = 0), sulphoxide (n = 1), or sulphone (n = 2) to a different such group;

(ii) removing the carboxyl-blocking group if present;

(iii) converting the free penicillanic acid into a pharmaceutically acceptable salt or ester thereof.

27. A process as claimed in claim 26 wherein the reducing agent is a trialkyl tin hydride.

28. A process as claimed in claim 27 wherein the reducing agent is tri-*n*-butyl tin hydride.

29. A process as claimed in claim 26 wherein the reducing agent is triphenyl tin hydride.

30. A process for the preparation of an in-vivo hydrolysable ester of 6-$\beta$-bromopenicillanic acid when substantially free from the corresponding in-vivo hydrolysable ester of 6-$\alpha$-bromopenicillanic acid which process comprises preparing a mixture of isomeric in-vivo hydrolysable esters of 6$\alpha$- and 6$\beta$-bromopenicillanic acids and thereafter chromatographically separating the mixture of isomers into fractions and recovering the in-vivo hydrolysable ester of 6-$\beta$-bromopenicillanic acid from a fraction containing said in-vivo hydrolysable ester of 6-$\beta$-bromopenicillanic acid substantially free from the in-vivo hydrolysable ester of a 6-$\alpha$-bromopenicillanic acid.

**Revendications pour les Etats contractants: BE CH IT NL SE**

1. Composé de formule (I):

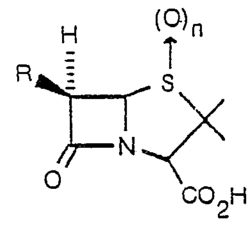

(I)

ou sel ou ester pharmaceutiquement acceptable de ce composé, formule dans laquelle R est un groupe alcoylthio en $C_{1-6}$, alcoyle en $C_{1-6}$ au alcoyle en $C_{1-6}$ substitué par un groupe phényle, carboxy, alcoxy($C_{1-6}$) carbonyle, hydroxy ou alcoylthio en $C_{1-6}$, et n est égal à 1 ou 2, à la condition que R ne soit pas un alcoyle en $C_{1-6}$ quand n est égal à 1.

2. Composé suivant la revendication 1, caractérisé en ce que R est un groupe méthylthio ou éthylthio.

3. Composé suivant la revendication 1, caractérisé en ce que R est un groupe méthyle, éthyle, n-propyle, isopropyle, n-butyle, sec-butyle ou isobutyle.

4. Composé suivant la revendication 1, caractérisé en ce que R est un groupe benzyle, phénétyle ou phénylpropyle.

5. Composé suivant la revendication 1, caractérisé en ce que R est un groupe carboxyméthyle, carboxyéthyle, carboxypropyle, méthoxycarbonylméthyle, méthoxycarbonyléthyle, éthoxycarbonylméthyle ou éthoxycarbonyléthyle.

6. Composé suivant la revendication 1, caractérisé en ce que R est un groupe hydroxyméthyle, 1-hydroxyéthyle, 1-hydroxy-n-propyle, 2-hydroxy-n-propyle, 3-hydroxy-n-propyle, 1-hydroxy-isopropyle ou 2-hydroxyisopropyle.

7. Composé suivant la revendication 1, caractérisé en ce que R est un groupe méthylthiométhyle ou méthylthioéthyle.

8. Composé suivant l'une quelconque des revendications 1 à 7, caractérisé en ce que n est égal à 2.

9. Composé de formule (I):

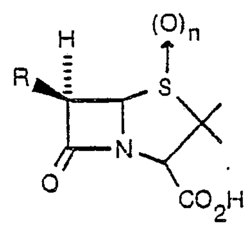

(I)

ou sel pharmaceutiquement acceptable ou ester hydrolysable in-vivo de ce composé, formule dans laquelle R est un substituant fluoro, bromo, chloro ou iodo, et n est égal à zéro, 1 ou 2, à la condition que lorsque R est un substituant bromo, ou chloro et n est égal à zéro, le composé ne soit pas contaminé par plus de 10% p/p du composé 6 β-correspondant.

10. Composé suivant la revendication 9, caractérisé en ce que R est un substituant iodo.

11. Composé suivant la revendication 9, caractérisé en ce que R est un substituant bromo.

12. Composé suivant la revendication 9, caractérisé en ce que R est un substituant chloro.

13. Composé suivant l'une quelconque des revendications 9 à 12, caractérisé en ce que n est égal à zéro.

14. Composé suivant l'une quelconque des revendications 1 à 13, sous la forme d'un sel de métal alcalin ou de métal alcalino-terreux.

15. Composé suivant l'une quelconque des revendications 1 à 14, sous la forme d'un sel de sodium ou de potassium.

16. Composé suivant l'une quelconque des revendications 1 à 13, sous la forme d'un ester hydrolysable in-vivo.

17. Composé suivant l'une quelconque des revendications 1 à 13 ou 16, caractérisé en ce que le groupe ester hydrolysable in-vivo est un groupe acétoxyméthyle, pivaloyloxyméthyle, α-acétoxyéthyle, α-acétoxybenzyle, α-pivaloyloxyéthyle, éthoxycarbonylméthyle, α-éthoxycarbonyléthyle, phtalidyle, 5,6-diméthoxyphtalide, diméthylaminométhyle, diméthylaminoéthyle, diéthylaminométhyle, diéthylaminoéthyle, N-phtalimidométhyle ou méthoxyméthyle.

18. Composé suivant la revendication 1, formé par le 1,1-dioxo-6 β-méthylpénicillanate de sodium.

19. Composé suivant la revendication 9, formé par le 6 β-bromopénicillanate de pivaloyloxyméthyle lorsqu'il n'est pas contaminé par plus de 10% du composé 6 α-correspondant.

20. Composé suivant la revendication 9, formé par le 6 β-bromopénicillanate de sodium lorsqu'il n'est pas contaminé par plus de 10% du composé 6 α-correspondant.

21. Composé suivant la revendication 9, formé par le 6 β-bromopénicillanate de sodium cristallin.

22. Composition pharmaceutique renfermant une pénicilline ou une céphalosporine, un véhicule ou excipient pharmaceutiquement acceptable et un composé de formule (I):

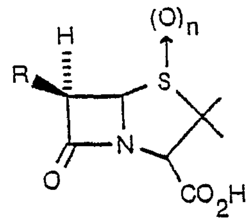

(I)

ou bien un sel pharmaceutiquement acceptable ou un ester hydrolysable in-vivo de ce composé, formule dans laquelle R est un halogène ou un groupe alcoylthio en $C_{1-6}$, alcoyle en $C_{1-6}$ ou alocyle en $C_{1-6}$ substitué par un groupe phényle, carboxy, alcoxy($C_{1-6}$)carbonyle, hydroxy ou alcoylthio en $C_{1-6}$, et n est égal à 0, 1 ou 2, à la condition que lorsque R est un bromo et n est égal à zéro, le composé ne soit pas contaminé par plus de 10% p/p du composé 6-correspondant.

23. Composition pharmaceutique renfermant un composé suivant l'une quelconque des revendication 1 à 21 et un véhicule ou excipient pharmaceutiquement acceptable.

24. Composition pharmaceutique renfermant un composé suivant l'une quelconque des revendications 1 à 21, une pénicilline ou une céphalosporine et un véhicule ou excipient pharmaceutiquement acceptable.

25. Composé suivant l'une quelconque des revendications 1 à 21, destiné à être utilisé pour le traitement des infections bactériennes.

26. Procédé pour la préparation d'un dérivé des pénicillines de formule (I):

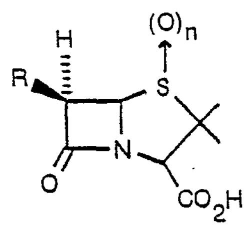

(I)

ou d'un sel ou ester pharmaceutiquement acceptable de celui-ci, formule dans laquelle R représente de l'hydrogène, un halogène ou un groupe alcoylthio en $C_{1-6}$, alcoyle en $C_{1-6}$ ou alcoyle substitué par un groupe phényle, carboxy, alcoxy ($C_{1-6}$)carbonyle, hydroxy ou alcoylthio en $C_{1-6}$, et n est égal à zéro, 1 ou 2, ce procédé consistant à traiter un composé de formule (V):

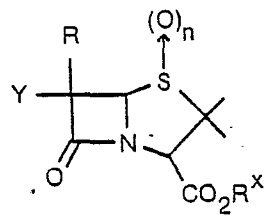

(V)

dans laquelle (a) R est tel que défini dans le cas de la formule (I) et Y représente un groupe isocyano ou un halogène, ou (b) R est un substituant chloro et Y est un groupe arylsélényle; $R^x$ représente de l'hydrogène ou un groupe de blocage du carboxyle et n est égal à zéro, 1 ou 2; avec un hydrure de triaryl-étain, un dihydrure de dialcoyl-étain ou un hydrure de trialcoyl-étain, puis facultativement à effectuer un ou plusieurs des stades opératoires ci-après:

(i) conversion d'un sulfure (n=0), d'un sulfoxyde (n=1) ou d'une sulfone (n=2) en un groupe de ce type différent;

(ii) élimination du groupe de blocage du carboxyle s'il est présent;

(iii) conversion de l'acide pénicillanique libre en un sel ou ester pharmaceutiquement acceptable de celui-ci.

27. Procédé suivant la revendication 26, caractérisé en ce que l'agent réducteur est un hydrure de trialcoyl-étain.

28. Procédé suivant la revendication 27, caractérisé en ce que l'agent réducteur est un hydrure de tri-n-butyl-étain.

29. Procédé suivant la revendication 26, caractérisé en ce que l'agent réducteur est de l'hydrure de triphényl-étain.

30. Procédé pour la préparation d'un ester hydrolysable in-vivo d'acide 6 $\beta$-bromopénicillanique ne contenant sensiblement pas d'ester hydrolysable in-vivo correspondant d'acide 6 $\alpha$-bromopénicillanique, ce procédé consistant à préparer un mélange des esters hydrolysables in-vivo isomères des acides 6 $\alpha$- et 6 $\beta$-bromopénicillaniques, puis à séparer par voie chromatographique le mélange d'isomères en ses fractions et à récupérer l'ester hydrolysable in-vivo de l'acide 6 $\beta$-bromopénicillanique à partir d'une fraction contenant cet ester hydrolysable in-vivo d'acide 6 $\beta$-bromopénicillanique ne contenant sensiblement pas d'ester hydrolysable in-vivo d'acide 6 $\alpha$-bromopénicillanique.

**Revendications pour les Etats contractants: DE FR GB**

1. Composé de formule (I):

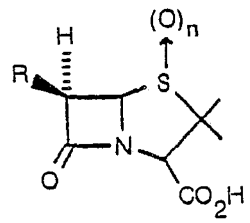

(I)

ou sel ou ester pharmaceutiquement acceptable de ce composé, formule dans laquelle R est un groupe alcoylthio en $C_{1-6}$, alcoyle en $C_{1-6}$ ou alcoyle en $C_{1-6}$ substitué par un groupe phényle, carboxy, alcoxy($C_{1-6}$)carbonyle, hydroxy ou alcoylthio en $C_{1-6}$, et n est égal à 1 ou 2, à la condition que R ne soit pas un alcoyle en $C_{1-6}$ quand n est égal à 1.

2. Composé suivant la revendication 1, caractérisé en ce que R est un groupe méthylthio ou éthylthio.

3. Composé suivant la revendication 1, caractérisé en ce que R est un groupe méthyle, éthyle, n-propyle, iso-propyle, n-butyle, sec-butyle ou isobutyle.

4. Composé suivant la revendication 1, caractérisé en ce que R est un groupe benzyle, phénétyle ou phénylpropyle.

5. Composé suivant la revendication 1, caractérisé en ce que R est un groupe carboxyméthyle, carboxyéthyle, carboxypropyle, méthoxycarbonylméthyle, méthoxycarbonyléthyle, éthoxycarbonylméthyle ou éthoxycarbonyléthyle.

6. Composé suivant la revendication 1, caractérisé en ce que R est un groupe hydroxyméthyle, 1-hydroxyéthyle, 1-hydroxy-n-propyle, 2-hydroxy-n-propyle, 3-hydroxy-n-propyle, 1-hydroxy-isopropyle ou 2-hydroxyisopropyle.

7. Composé suivant la revendication 1, caractérisé en ce que R est un groupe méthylthiométhyle ou méthylthioéthyle.

8. Composé suivant l'une quelconque des revendications 1 à 7, caractérisé en ce que n est égal à 2.

9. Composé de formule (I):

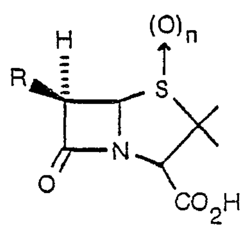

(I)

ou sel pharmaceutiquement acceptable ou ester hydrolysable in-vivo de ce composé, formule dans laquelle R est un substituant fluoro, bromo, chloro ou iodo, et n est égal à zéro, 1 ou 2, à la condition que lorsque R est un substituant bromo et n est égal à zéro ou 2, ou bien R est un substituant chloro et n est

égal à zéro, alors dans chaque cas le composé n'est pas contaminé par plus de 10% p/p du composé 6 $\alpha$-correspondant.

10. Composé suivant la revendication 9, caractérisé en ce que R est un substituant iodo.

11. Composé suivant la revendication 9, caractérisé en ce que R est un substituant bromo.

12. Composé suivant la revendication 9, caractérisé en ce que R est un substituant chloro.

13. Composé suivant l'une quelconque des revendications 9 à 12, caractérié en ce que n est égal à zéro.

14. Composé suivant l'une quelconque des revendications 1 à 13, sous la forme d'un sel de métal alcalin ou de métal alcalino-terreux.

15. Composé suivant l'une quelconque des revendications 1 à 14, sous la forme d'un sel de sodium ou de potassium.

16. Composé suivant l'une quelconque des revendications 1 à 13, sous la forme d'un ester hydrolysable in-vivo.

17. Composé suivant l'une quelconque des revendications 1 à 13 ou 16, caractérisé en ce que le groupe ester hydrolysable in-vivo est un groupe acétoxyméthyle, pivaloyloxyméthyle, $\alpha$-acétoxyéthyle, $\alpha$-acétoxybenzyle, $\alpha$-pivaloyloxyéthyle, éthoxycarbonyloxyméthyle, $\alpha$-éthoxycarbonyléthyle, phtalidyle, 5,6-diméthoxyphtalidyle, diméthylaminométhyle, diméthylaminoéthyle, diéthylaminométhyle, diéthylaminoéthyle, N-phtalimidométhyle ou méthoxyméthyle.

18. Composé suivant la revendication 1, formé par le 1,1-dioxo-6 $\beta$-méthylpénicillanate de sodium.

19. Composé suivant la revendication 9, formé par le 6 $\beta$-bromopénicillanate de pivaloyloxyméthyle lorsqu'il n'est pas contaminé par plus de 10% du composé 6 $\alpha$-correspondant.

20. Composé suivant la revendication 9, formé par le 6 $\beta$-bromopénicillanate de sodium lorsqu'il n'est pas contaminé par plus de 10% du composé 6 $\alpha$-correspondant.

21. Composé suivant la revendication 9, formé par le 6 $\beta$-bromopénicillanate de sodium cristallin.

22. Composition pharmaceutique renfermant une pénicilline ou une céphalosporine, un véhicule ou excipient pharmaceutiquement acceptable et un composé de formule (I):

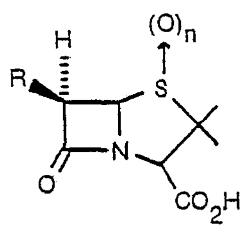

(I)

ou un sel pharmaceutiquement acceptable ou ester hydrolysable in-vivo de celui-ci, formule dans laquelle R est un halogène, ou un groupe alcoylthio en $C_{1-6}$, alcoyle en $C_{1-6}$ ou alcoyle en $C_{1-6}$ substitué par un groupe phényle, carboxy, alcoxy $(C_{1-6})$carbonyle, hydroxy ou alcoylthio en $C_{1-6}$ et n est égal à zéro, 1 ou 2, à la condition que lorsque R est un substituant bromo, n soit égal à zéro ou 2 et le composé ne soit pas contaminé par plus de 10% p/p du composé 6 $\alpha$-correspondant.

23. Composition pharmaceutique renfermant un composé suivant l'une quelconque des revendications 1 à 21 et un véhicule ou excipient pharmaceutiquement acceptable.

24. Composition pharmaceutique renfermant un composé suivant l'une quelconque des revendications 1 à 21, une pénicilline ou une céphalosporine et un véhicule ou excipient pharmaceutiquement acceptable.

25. Composé suivant l'une quelconque des revendications 1 à 21, destiné à être utilisé pour le traitement des infections bactériennes.

26. Procédé pour la préparation d'un dérivé des pénicillines de formule (I):

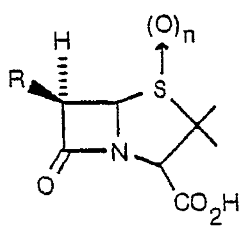

(I)

ou d'un sel ou ester pharmaceutiquement acceptable de celui-ci, formule dans laquelle R représente de l'hydrogène, un halogène ou un groupe alcoylthio en $C_{1-6}$, alcoyle en $C_{1-6}$ ou alcoyle substitué par un groupe phényle, carboxy, alcoxy $(C_{1-6})$carbonyle, hydroxy ou alcoylthio en $C_{1-6}$, et n est égal à zéro, 1 ou 2, ce procédé consistant à traiter un composé de formule (V):

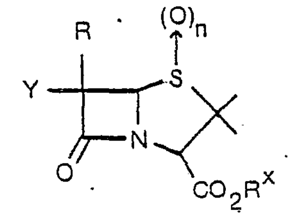

(V)

dans laquelle (a) R est tel que défini dans le cas de la formule (I) et Y représente un groupe isocyano ou un halogène, ou (b) R est un substituant chloro et Y est un groupe arylsélényle; $R^x$ représente de l'hydrogène ou un groupe de blocage du carboxyle et n est égal à zéro, 1 ou 2; avec un hydrure de triaryl-étain, un dihydrure de dialcoyl-étain ou un hydrure de trialcoyl-étain, puis facultativement à effectuer un ou plusieurs des stades opératoires ci-après:

(i) conversion d'un sulfure (n=0), d'un sulfoxyde (n=1) ou d'une sulfone (n=2) en un groupe de ce type différent;

(ii) élimination du groupe de blocage du carboxyle s'il est présent;

(iii) conversion de l'acide péncillanique libre en un sel ou ester pharmaceutiquement acceptable de celui-ci.

27. Procédé suivant la revendication 26, caractérisé en ce que l'agent réducteur est un hydrure de trialcoyl-étain.

28. Procédé suivant la revendication 27, caractérisé en ce que l'agent réducteur est un hydrure de tri-n-butyl-étain.

29. Procédé suivant la revendication 26, caractérisé en ce que l'agent réducteur est de l'hydrure de tri-phényl-étain.

30. Procédé pour la préparation d'un ester hydrolysable in-vivo d'acide 6 $\beta$-bromopénicillanique ne contenant sensiblement pas d'ester hydrolysable in-vivo correspondant d'acide 6 $\alpha$-bromopénicillanique, ce procédé consistant à préparer un mélange des esters hydrolysables in-vivo isomères des acides 6 $\alpha$- et 6 $\beta$-bromopénicillaniques, puis à séparer par voie chromatographique le mélange d'isomères en ses fractions et a récupérer l'ester hydrolysable in-vivo de l'acide 6 $\beta$-bromopénicillanique à partir d'une fraction contenant cet ester hydrolysable in-vivo d'acide 6 $\beta$-bromopénicillanique ne contenant sensiblement pas d'ester hydrolysable in-vivo d'acide 6 $\alpha$-bromopénicillanique.

## Patentansprüche für die Vertragsstaaten: BE CH IT NL SE

1. Eine Verbindung der Formel (I)

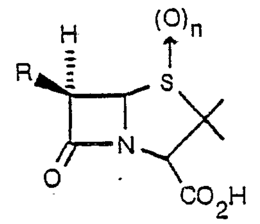

(I)

oder ein pharmazeutisch verträgliches Salz oder ein Ester — derselben, wobei R $C_{1-6}$-Alkylthio, $C_{1-6}$-Alkyl oder $C_{1-6}$-Alkyl, substituiert durch Phenyl, Carboxy, $C_{1-6}$-Alkoxycarbonyl, Hydroxy oder $C_{1-6}$-Alkylthio, ist und n 1 oder 2 bedeutet, mit der Maßgabe, daß R kein $C_{1-6}$-Alkyl ist, wenn n 1 bedeutet.

2. Eine Verbindung wie in Anspruch 1 beansprucht, in welcher R eine Methylthio- oder Athylthiogruppe ist.

3. Eine Verbindung wie in Anspruch 1 beansprucht, in welcher R Methyl, Äthyl, n-Propyl, Isopropyl, n-Butyl, sec.Butyl oder Isobutyl ist.

4. Eine Verbindung wie in Anspruch 1 beansprucht, in welcher R Benzyl, Phenäthyl oder Phenylpropyl ist.

5. Eine Verbindung wie in Anspruch 1 beansprucht, in welcher R Carboxymethyl, Carboxyäthyl, Carboxypropyl, Methoxycarbonylmethyl, Methoxycarbonyläthyl, Äthoxycarbonylmethyl oder Äthoxycarbonyläthyl ist.

6. Eine Verbindung wie in Anspruch 1 beansprucht, in welcher R Hydroxymethyl, 1-Hydroxyäthyl, 1-Hydroxy-n-propyl, 2-Hydroxy-n-propyl, 3-Hydroxy-n-propyl, 1-Hydroxy-isopropyl oder 2-Hydroxyisopropyl ist.

7. Eine Verbindung wie in Anspruch 1 beansprucht, in welcher R Methylthiomethyl oder Methylthioäthyl ist.

8. Eine Verbindung wie in irgendeinem der Ansprüche 1 bis 7 beansprucht, in welcher n 2 bedeutet.

9. Eine Verbindung der Formel(I):

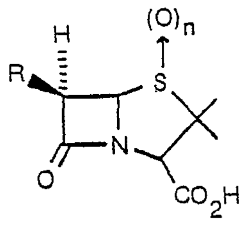

(I)

oder ein pharmazeutisch verträgliches Salz oder ein in-vivo hydrolysierbarer Ester derselben, wobei R Fluor, Brom, Chlor oder Iod ist und n 0, 1 oder 2 bedeutet, mit der Maßgabe, daß, wenn R Brom oder Chlor und n 0 bedeuten, die Verbindung durch nicht mehr als 10 Gewichtsprozent der entsprechenden 6 $\alpha$-Verbindung verunreinigt ist.

10. Eine Verbindung wie in Anspruch 9 beansprucht, in welcher R Iod ist.

11. Eine Verbindung wie in Anspruch 9 beansprucht, in welcher R Brom ist.

12. Eine Verbindung wie in Anspruch 9 beansprucht, in welcher R Chlor ist.

13. Eine Verbindung wie in irgendeinem der Ansprüche 9 bis 12 beansprucht, wobei n 0 bedeutet.

14. Eine Verbindung wie in irgendeinem der Ansprüche 1 bis 13 beansprucht, in Form eines Alkalimetall- oder eines Erdalkalimetallsalzes.

15. Eine Verbindung wie in irgendeinem der Ansprüche 1 bis 14 beansprucht, in Form eines Natrium- oder Kaliumsalzes.

16. Eine Verbindung wie in irgendeinem der Ansprüche 1 bis 13 beansprucht, in Form eines in-vivo hydrolysierbaren Esters.

17. Eine Verbindung wie in irgendeinem der Ansprüche 1 bis 13 oder 16 beansprucht, in welcher die in-vivo hydrolysierbare Estergruppe Acetoxymethyl, Pivaloyloxymethyl, $\alpha$-Acetoxyäthyl, $\alpha$-Acetoxybenzyl, $\alpha$-Pivaloyloxyäthyl, Äthoxycarbonyloxymethyl, $\alpha$-Äthoxycarbonyläthyl, Phthalidyl, 5,6-Dimethoxyphthalidyl, Dimethylaminomethyl, Dimethylaminoäthyl, Diäthylaminomethyl, Diäthylaminoäthyl, N-Phthalimidomethyl oder Methoxymethyl ist.

18. Eine Verbindung wie in Anspruch 1 beansprucht, welche Natrium-1,1-dioxo-6$\beta$-methylpenicillanat ist.

19. Eine Verbindung wie in Anspruch 9 beansprucht, welche Pivaloyloxymethyl-6$\beta$-brompenicillanat ist, wenn sie durch nicht mehr als 10% der entsprechenden 6$\alpha$-Verbindung verunreinigt ist.

20. Eine Verbindung wie in Anspruch 9 beansprucht, welche Natrium-6$\beta$-brompenicillanat ist, wenn sie durch nicht mehr als 10% der entsprechenden 6$\alpha$-Verbindung verunreinigt ist.

21. Eine Verbindung wie in Anspruch 9 beansprucht, welche ein kristallines Natrium-6$\beta$-bromopenicillanat ist.

22. Eine pharmazeutische Zusammensetzung, welcher Penicillin oder Cephalosporin, einen Pharmazeutisch verträglichen Träger und eine Verbindung Formel(I):

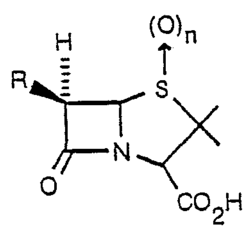

(I)

oder ein pharmazeutisch verträgliches Salz oder einen in-vivo hydrolysierbaren Ester derselben enthält, wobei R ein Halogen, $C_{1-6}$-Alkylthio, $C_{1-6}$-Alkyl oder $C_{1-6}$-Alkyl, substituiert durch Phenyl, Carboxy, $C_{1-6}$-Alkoxycarbonyl, Hydroxy oder $C_{1-6}$-Alkylthio, und n 0, 1 oder 2 ist, mit der Maßgabe, daß, wenn R Brom und n 0 bedeutet, die Verbindung durch nicht mehr als 10 Gewichtsprozent der entsprechenden 6$\alpha$-Verbindung verunreinigt ist.

23. Eine pharmazeutische Zusammensetzung, welche eine Verbindung wie in irgendeinem der Ansprüche 1 bis 21 beansprucht und einen pharmazeutisch verträglichen Träger umfaßt.

24. Eine pharmazeutische Zusammensetzung, welche eine Verbindung wie in irgendeinem der Ansprüche 1 bis 21 beansprucht, ein Penicillin oder Cephalosporin und einen pharmazeutisch verträglichen Träger umfaßt.

25. Eine Verbindung wie in irgendeinem der Ansprüche 1 bis 21 beansprucht zur Verwendung bei der Behandlung von bakteriellen Infektionen.

26. Ein Verfahren zur Herstellung eines Penicillinderivats der Formel(I):

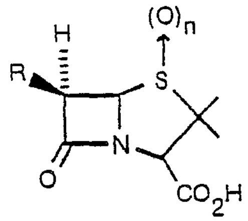

(I)

oder eines pharmazeutisch verträglichen Salzes oder Esters derselben, wobei R Wasserstoff, ein Halogen, $C_{1-6}$-Alkylthio, $C_{1-6}$-Alkyl oder Alkyl, substituiert durch Phenyl, Carboxy, $C_{1-6}$-Alkoxycarbonyl, Hydroxy oder $C_{1-6}$-Alkylthio, ist und n 0, 1 oder 2 bedeutet, welches Verfahren das Behandeln einer Verbindung der Formel(V):

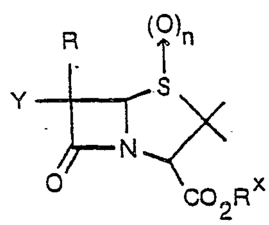

(V)

in welcher entweder (a) R wie in bezug auf Formel (I) definiert ist und Y Isocyano oder ein Halogen ist, oder (b) R Chlor und Y Arylselenyl ist, $R^x$ Wasserstoff oder eine Carboxyl-Hemmgruppe darstellt und n 0, 1 oder 2 ist, mit einem Triaryl-Zinnhydrid, einem Dialkyl-Zinndihydrid oder einem Trialkyl-Zinnhydrid, und anschliessend gewünschtenfalls die Durchführung eines oder mehrerer der folgenden Schritte umfaßt:

(i) Umwandeln eines Sulfids (n=0), Sulfoxids (n=1), oder Sulfons (n=2) in eine andere Gruppe dieser Art;

(ii) Entfernung der Carboxyl-Hemmgruppe, falls vorhanden;

(iii) Umwandeln der freien Penicillinsäure in ein pharmazeutisch verträgliches Salz oder einen Ester derselben.

27. Ein Verfahren wie in Anspruch 26 beansprucht, wobei das Reduktionsmittel ein Trialkyl-Zinnhydrid ist.

28. Ein Verfahren wie in Anspruch 27 beansprucht, wobei das Reduktionsmittel Tri-n-butyl-Zinnhydrid ist.

29. Ein Verfahren wie in Anspruch 26 beansprucht, wobei das Reduktionsmittel Triphenyl-Zinnhydrid ist.

30. Ein Verfahren zur Herstellung eines in-vivo hydrolysierbaren Esters der 6-$\beta$-Brompenicillansäure, wenn diese im wesentlichen frei ist von dem entsprechenden in-vivo hydrolysierbaren Ester der 6-$\alpha$-Brompenicillansäure, welches Verfahren die Herstellung eines Mischung der isomeren in-vivo hydrolysierbaren Ester der 6$\alpha$- und 6$\beta$-Bromopenicillansäuren, das anschließende chromatographische Auftrennen der Mischung von Isomeren in Fraktionen und die Wiedergewinnung des in-vivo hydroysierbaren Esters der 6-$\beta$-Brompenicillansäure aus einer Fraktion, enthaltend den genannten in-vivo hydrolysierbaren Ester der 6-$\beta$-Brompenicillansäure, welche im wesentlichen frei ist von dem in-vivo hydrolysierbaren Ester der 6-$\alpha$-Brompenicillansäure, umfaßt.

**Patentansprüche für die Vertragsstaaten: DE FR GB**

1. Eine Verbindung der Formel (I)

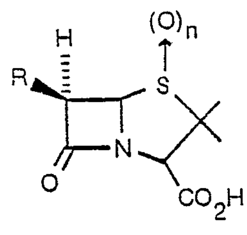

(I)

oder ein pharmazeutisch verträgliches Salz oder ein Ester — derselben, wobei R $C_{1-6}$-Alkylthio, $C_{1-6}$-Alkyl oder $C_{1-6}$-Alkyl, substituiert durch Phenyl, Carboxy, $C_{1-6}$-Alkoxycarbonyl, Hydroxy oder $C_{1-6}$-Alkylthio, ist und n 1 oder 2 bedeutet, mit der Maßgabe, daß R kein $C_{1-6}$-Alkyl ist, wenn n 1 bedeutet.

2. Eine Verbindung wie in Anspruch 1 beansprucht, in welcher R eine Methylthio- oder Äthylthiogruppe ist.

3. Eine Verbindung wie in Anspruch 1 beansprucht, in welcher R Methyl, Äthyl, n-Propyl, Isopropyl, n-Butyl-, sec.Butyl oder Isobutyl ist.

4. Eine Verbindung wie in Anspruch 1 beansprucht, in welcher R Benzyl, Phenäthyl oder Phenylpropyl ist.

5. Eine Verbindung wie in Anspruch 1 beansprucht, in welcher R Carboxymethyl, Carboxyäthyl, Carboxypropyl, Methoxycarbonylmethyl, Methoxycarbonyläthyl, Äthoxycarbonylmethyl oder Äthoxycarbonyläthyl ist.

6. Eine Verbindung wie in Anspruch 1 beansprucht, in welcher R Hydroxymethyl, 1-Hydroxyäthyl, 1-Hydroxy-n-propyl, 2-Hydroxy-n-propyl, 3-Hydroxy-n-propyl, 1-Hydroxy-isopropyl oder 2-Hydroxyisopropyl ist.

7. Eine Verbindung wie in Anspruch 1 beansprucht, in welcher R Methylthiomethyl oder Methylthioäthyl ist.

8. Eine Verbindung wie in irgendeinem der Ansprüche 1 bis 7 beansprucht, in welcher n 2 bedeutet.

9. Eine Verbindung der Formel(I):

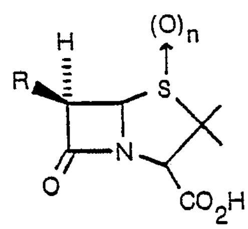

(I)

oder ein pharmazeutisch verträgliches Salz oder ein in-vivo hydrolysierbarer Ester derselben, wobei R Fluor, Brom, Chlor oder Iod ist und n 0, 1 oder 2 bedeutet, mit der Maßgabe, daß, wenn R Brom und n 0 oder 2 ist oder R Chlor ist und n 0 bedeutet, dann in jedem Fall die Verbindung durch nicht mehr als 10 Gewichtsprozent der entsprechenden 6-$\alpha$-Verbindung verunreinigt ist.

10. Eine Verbindung wie in Anspruch 9 beansprucht, in welcher R Iod ist.

11. Eine Verbindung wie in Anspruch 9 beansprucht, in welcher R Brom ist.

12. Eine Verbindung wie in Anspruch 9 beansprucht, in welcher R Chlor ist.

13. Eine Verbindung wie in irgendeinem der Ansprüche 9 bis 12 beansprucht, wobei n 0 bedeutet.

14. Eine Verbindung wie in irgendeinem der Ansprüche 1 bis 13 beansprucht, in Form eines Alkalimetall- oder eines Erdalkalimetallsalzes.

15. Eine Verbindung wie in irgendeinem der Ansprüche 1 bis 14 beansprucht, in Form eines Natrium- oder Kaliumsalzes.

16. Eine Verbindung wie in irgendeinem der Ansprüche 1 bis 13 beansprucht, in Form eines in-vivo hydrolysierbaren Esters.

17. Eine Verbindung wie in irgendeinem der Ansprüche 1 bis 13 oder 16 beansprucht, in welcher die in-vivo hydrolysierbare Estergruppe Acetoxymethyl, Pivaloyloxymethyl, $\alpha$-Acetoxyäthyl, $\alpha$-Acetoxybenzyl, $\alpha$-Pivaloyloxyäthyl, Athoxycarbonyloxymethyl, $\alpha$-Äthoxycarbonyläthyl, Phthalidyl, 5,6-Dimethoxyphthalidyl, Dimethylaminomethyl, Dimethylaminoäthyl, Diäthylaminomethyl, Diäthylaminoäthyl, N-Phthalimidomethyl oder Methoxymethyl ist.

18. Eine Verbindung wie in Anspruch 1 beansprucht, welche Natrium-1,1-dioxo-6$\beta$-methylpenicillanat ist.

19. Eine Verbindung wie in Anspruch 9 beansprucht, welche Pivaloyloxymethyl-6β-brompenicillanat ist, wenn sie durch nicht mehr als 10% der entsprechenden 6α-Verbindung verunreinigt ist.

20. Eine Verbindung wie in Anspruch 9 beansprucht, welche Natrium-6β-brompenicillanat ist, wenn sie durch nicht mehr als 10% der entsprechenden 6α-Verbindung verunreinigt ist.

21. Eine Verbindung wie in Anspruch 9 beansprucht, welche ein kristallines Natrium-6β-brompenicillanat ist.

22. Eine pharmazeutische Zusammensetzung, welche Penicillin oder Cephalosporin, einen pharmazeutisch verträglichen Träger und eine Verbindung Formel(I):

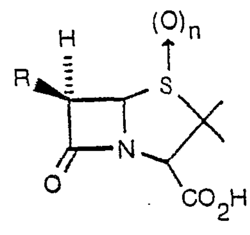

(I)

oder ein pharmazeutisch verträgliches Salz oder einen in-vivo hydrolysierbaren Ester derselben enthält, wobei R ein Halogen, $C_{1-6}$-Alkylthio, $C_{1-6}$-Alkyl oder $C_{1-6}$-Alkyl, substituiert durch Phenyl, Carboxy, $C_{1-6}$-Alkoxycarbonyl, Hydroxy oder $C_{1-6}$-Alkylthio, und n 0, 1 oder 2 ist, mit der Maßgabe, daß, wenn R Brom bedeutet und n 0 oder 2 ist, die Verbindung durch nicht mehr als 10 Gewichtsprozent der entsprechenden 6α-Verbindung verunreinigt ist. .

23. Eine pharmazeutische Zusammensetzung, welche eine Verbindung wie in irgendeinem der Ansprüche 1 bis 21 beansprucht und einem pharmazeutisch verträglichen Träger umfaßt.

24. Eine pharmazeutische Zusammensetzung, welche eine Verbindung wie in irgendeinem der Anspruch 1 bis 21 beansprucht, ein Penicillin oder Cephalosporin und einen pharmazeutisch verträglichen Träger umfaßt.

25. Eine Verbindung wie in irgendeinem der Ansprüche 1 bis 21 beansprucht zur Verwendung bei der Behandlung von bakteriellen Infektionen.

26. Ein Verfahren zur Herstellung eines Penicillinderivats der Formel(I):

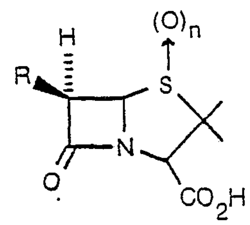

(I)

oder eines pharmazeutisch verträglichen Salzes oder Esters derselben, wobei R Wasserstoff, ein Halogen, $C_{1-6}$-Alkylthio, $C_{1-6}$-Alkyl oder Alkyl, substituiert durch Phenyl, Carboxy, $C_{1-6}$-Alkoxycarbonyl, Hydroxy oder $C_{1-6}$-Alkylthio, ist, und n 0, 1 oder 2 bedeutet, welches Verfahren das Behandeln einer Verbindung der Formel(V):

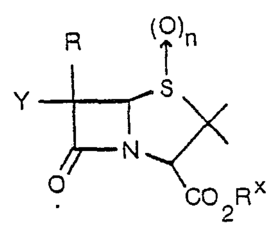

(V)

in welcher entweder (a) R wie in bezug auf Formel(I) definiert ist und Y Isocyano oder ein Halogen ist, oder (b) R Chlor und Y Arylselenyl ist, $R^x$ Wasserstoff oder eine Carboxyl-Hemmgruppe darstellt und n 0, 1 oder 2 ist, mit einem Triaryl-Zinnhydrid, einem Dialkyl-Zinndihydrid oder einem Trialkyl-Zinnhydrid und anschliessend gewünschtenfalls die Durchführung eines oder mehrerer der folgenden Schritte umfaßt.

(i) Umwandeln eines Sulfids (n=0), Sulfoxids (n=1, oder Sulfons (n=2) in eine andere Gruppe dieser Art;

(ii) Entfernen der Carboxyl-Hemmgruppe, falls vorhanden;

(iii) Umwandeln der freien Penicillinsäure in ein pharmazeutisch verträgliches Salz oder einen Ester derselben.

27. Ein Verfahren wie in Anspruch 26 beansprucht, wobei das Reduktionsmittel ein Trialkyl-Zinnhydrid ist.

28. Ein Verfahren wie in Anspruch 27 beansprucht, wobei das Reduktionsmittel Tri-n-butyl-Zinnhydrid ist.

29. Ein Verfahren wie in Anspruch 26 beansprucht, wobei das Reduktionsmittel Triphenyl-Zinnhydrid ist.

30. Ein Verfahren zur Herstellung eines in-vivo hydrolysierbaren Esters der 6-β-Brompenicillansäure, wenn diese im wesentlichen frei ist von dem entsprechenden in-vivo hydrolysierbaren Ester der 6-α-Brompenicillansäure, welches Verfahren die Herstellung eines Mischung der isomeren in-vivo hydrolysierbaren Ester der 6 α- und 6 β-Brompenicillansäuren, das anschließende chromatographische Auftrennen der Mischung von Isomeren in Fraktionen und die Wiedergewinnung des in-vivo hydroysierbaren Esters der 6-β-Brompenicillansäure aus einer Fraktion, enthaltend den genannten in-vivo hydrolysierbaren Ester der 6-β-Brompenicillansäure, welche im wesentlichen frei ist von dem in-vivo hydrolysierbaren Ester der 6-α-Brompenicillansäure, umfaßt.